# EUROPEAN PATENT APPLICATION

(11) **EP 4 632 752 A1**
(43) Date of publication of application: **15.10.2025**
(21) Application number: 23901139.8
(22) Date of filing: 08.12.2023
(51) Int. Cl.: G16H 20/10, G16B 50/00, G16H 70/00, G16B 30/10, G16B 15/00, G16B 25/20

(54) **DEVICE AND METHOD FOR MANAGING RESEARCH AND DEVELOPMENT OF IN VITRO DIAGNOSTIC REAGENT PRODUCTS**

(30) Priority: 08.12.2022 KR 20220170746
(71) Applicant: Seegene, Inc., Seoul 05548 (KR)
(72) Inventor: KIM, Youngwook, Seoul 05548 (KR); LEE, Seungheon, Seoul 05548 (KR); JEON, Chang Ha, Seoul 05548 (KR)
(74) Representative: Viering, Jentschura & Partner mbB Patent- und Rechtsanwälte
(86) International application number: PCT/KR2023/020150
(87) International publication number: WO 2024/123122

(57) **Abstract**

The present invention relates to a device and a method for managing research and development of in vitro diagnostic reagent products, the device and the method providing a development process through a research and development management program provided to research and development companies that develop in vitro diagnostic reagent products, and allowing each of the research and development companies to develop reagent products through a development process according to a standardized policy such that all reagent products can be tested using the same in vitro diagnostic device.

## Description

### TECHNICAL FIELD

The present disclosure relates to a device and a method for managing research and development of in vitro diagnostic reagent products, and more particularly, to a device and method for managing research and development of in vitro diagnostic reagent products that provides a development process through a research and development management program provided to research and development companies that develop in vitro diagnostic reagent products, and allows each of the research and development companies to develop reagent products through a development process according to a standardized policy such that all reagent products may be tested using the same in vitro diagnostic device.

### BACKGROUND ART

In vitro diagnostics (IVD) refers to a technology that analyzes target objects such as blood, urine, or cells collected from the human body to diagnose health conditions. Such in vitro diagnostics includes immunodiagnosis, self-blood glucose measurement, molecular diagnostic technologies, etc.

Among these, the immunodiagnosis is a method that uses a specific antibody reaction to an antigen in analyzing a sample. A representative example of the immunodiagnosis is known as an enzyme-linked immunosorbent assay (ELISA). According to the ELISA, a current disease condition may be simply diagnosed. However, the ELISA is known to have slightly low sensitivity.

Molecular diagnostics is a method for diagnosing diseases by detecting and analyzing DNA or RNA which is a genetic information material. A representative example of the molecular diagnostics is known as polymerase chain reaction (PCR). In the PCR, a trace amount of specific DNA may be amplified into a large amount in a short period of time. The PCR has a higher sensitivity than the above-described immunodiagnosis. Recently, real-time polymerase chain reaction (real-time PCR) using a fluorescent probe is becoming widespread and is being used to diagnose various types of diseases.

In the in vitro diagnostics including the above-described immunodiagnosis and molecular diagnostics, reagent products for the in vitro diagnostics are classified as medical devices. Since the reagent products are the medical devices, the reagent products should be approved for sale in each country.

In this way, the in vitro diagnostic reagent products should be developed in various ways to diagnose various types of diseases using medical devices. However, in reality, research and development companies of various reagent products are competitively engaged in the development and production of reagent products for diagnosing well-known diseases. In addition, the reagent products developed by each research and development company should use dedicated testing equipment. Therefore, multiple testing equipment should be provided in order to test the reagent products of the plurality of research and development companies. For example, due to the incompatible nature of the in vitro diagnostic reagent products, particularly molecular diagnostic reagent products, a laboratory may require at least 20 essential pieces of equipment.

As a result, there is a problem that it is difficult to substantially perform various tests in primary and secondary hospitals where patients may easily access. As a result, patients are unable to perform in vitro diagnostic tests at the early stage of their disease, which also leads to problems in delaying proper medical examination and treatment.

### DETAILED DESCRIPTION OF THE DISCLOSURE

### TECHNICAL PROBLEMS

The present disclosure is directed to providing a device and method for managing research and development of in vitro diagnostic reagent products capable of developing customized products desired by patients with various diseases or medical institutions treating diseases.

In addition, the present disclosure is directed to providing a device and method for managing research and development of in vitro diagnostic reagent products capable of easily developing a variety of in vitro diagnostic reagent products required by research and development companies in various medical departments.

In addition, the present disclosure is directed to enabling various in vitro diagnostic reagent products developed and produced by research and development companies to be tested on single testing equipment.

In addition, the present disclosure is directed to providing a research and development management program in which a research and development process and a production process are implemented such that research and development companies may develop and produce various in vitro diagnostic reagent products in a standardized system.

In addition, the present disclosure is directed to providing a device and method for managing research and development of in vitro diagnostic reagent products, capable of preventing duplicate research and development by providing reference data for research and development extracted from research and development data and production data in the same format obtained by the same research and development process and production process of research and development companies to other research and development companies that require the reference data for research and development.

In addition, the present disclosure is directed to providing a device and method for managing research and development of in vitro diagnostic reagent products capable of enabling a virtuous cycle of expanding reagent development to meet increasing demand in the field of in vitro diagnostic tests, particularly molecular diagnostic tests and increasing demand through such expansion.

In addition, the present disclosure is directed to providing research and development management programs and production management programs to research and development companies in the same manner through a standardized device for managing research and development used in research and development and production of in vitro diagnostic reagent products, particularly molecular diagnostic reagent products to reduce costs required for research and development and lower prices of reagent products accordingly, thereby providing a wide range of testing opportunities to many patients.

### MEANS FOR SOLVING PROBLEMS

The above objective of the present invention is achieved by a device for managing research and development of an in vitro diagnostic reagent product, including: a memory that stores at least one instruction; and a processor, wherein the memory stores a research and development management program that is provided to research and development companies developing the in vitro diagnostic reagent product, wherein the at least one instruction, when executed by the processor, causes the research and development management program to: commonly provide the same research and development process to the research and development companies such that the research and development of the in vitro diagnostic reagent product is carried out using the same technology; and store in a database research and development data of each research and development company obtained using research and development equipment for the research and development of the in vitro diagnostic reagent product according to the same research and development process, and wherein the database is configured to allocate a storage space with the same structure to each of the research and development companies, and the research and development data of each of the research and development companies has data compatibility such that the research and development data is compared with each other.

The above objective of the present invention is achieved by a computer-implemented method for research and development of in vitro diagnostic reagent product which is performed in a device for managing research and development of in vitro diagnostic reagent product using a memory, a processor, and one or more programs stored in the memory and configured to be executed by the processor, wherein the device for managing research and development stores a research and development management program that is provided to research and development companies developing the in vitro diagnostic reagent product, wherein the research and development management program includes: commonly providing the same research and development process to the research and development companies such that the research and development of the in vitro diagnostic reagent product is executed using the same technology; and storing in a database the research and development data of each research and development company obtained using the research and development equipment for the research and development of the in vitro diagnostic reagent product according to the same research and development process, and wherein the database is configured to allocate a storage space with the same structure to each of the research and development companies, and the research and development data of each of the research and development companies has data compatibility such that the research and development data is compared with each other.

The above objective of the present invention is achieved by a computer-readable recording medium storing a computer program allowing research and development companies to manage research and development of in vitro diagnostic reagent product by a device for managing research and development of in vitro diagnostic reagent product, wherein the computer program performs: commonly providing the same research and development process to the research and development companies such that the research and development of the in vitro diagnostic reagent product is carried out using the same technology; and storing in a database the research and development data of each research and development company obtained using the research and development equipment for the research and development of the in vitro diagnostic reagent product according to the same research and development process, and wherein the database is configured to allocate a storage space with the same structure to each of the research and development companies, and the research and development data of each of the research and development companies has data compatibility such that the research and development data is compared with each other.

The above objective of the present invention is achieved by a computer program stored on a computer-readable recording medium for allowing research and development companies to manage research and development of in vitro diagnostic reagent product by a device for managing research and development of in vitro diagnostic reagent product, wherein the computer program is programmed to perform commonly providing the same research and development process to the research and development companies such that the research and development of the in vitro diagnostic reagent product is carried out using the same technology; and storing in a database the research and development data of each research and development company obtained using the research and development equipment for the research and development of the in vitro diagnostic reagent product according to the same research and development process, and wherein the database is configured to allocate a storage space with the same structure to each of the research and development companies, and the research and development data of each of the research and development companies has data compatibility such that the research and development data is compared with each other.

### EFFECTS OF INVENTION

According to one embodiment of the present disclosure, research and development companies participating in reagent development through a device for managing research and development of in vitro diagnostic reagent products may share research and development information with each other because they generate research and development data and production data in the same format, thereby providing a superior research and development environment compared to other companies that do not participate in the reagent development.

According to another embodiment of the present disclosure, by providing reference data for research and development extracted from research and development data and/or production data obtained from research and development and/or production activities of research and development companies to other research and development companies, it is possible to shorten the development period of reagent products.

According to another embodiment of the present disclosure, by shortening the development period of reagent products by using reference data for research and development provided by a device for managing research and development of in vitro diagnostic reagent products, it is possible to provide research and development companies with an environment in which they may develop a wider variety of reagent products.

According to another embodiment of the present disclosure, each in vitro diagnostic reagent product produced through research and development and production using the same technology, the same equipment, and the same raw materials based on the same research and development and production process may perform in vitro diagnostic tests using the same testing equipment, thereby having the effect of making diagnostic tests a part of daily life.

### BRIEF DESCRIPTION OF THE DRAWINGS

FIG. 1 is a configuration diagram illustrating an environment for developing in vitro diagnostic reagent products, including a device for managing research and development of in vitro diagnostic reagent products and research and development companies connected via a communication network, according to the present disclosure.
FIG. 2 is a configuration diagram conceptually illustrating a device connected to the device for managing research and development of in vitro diagnostic reagent products according to one embodiment of the present disclosure.
FIG. 3 is a block diagram exemplarily illustrating a configuration of the device for managing research and development of in vitro diagnostic reagent products according to the present disclosure.
FIG. 4 is a configuration diagram illustrating each stage of a process for developing in vitro diagnostic reagent products and data obtained through the process according to one embodiment of the present disclosure.
FIG. 5 is a block diagram exemplarily illustrating a concept of storing research and development data and production data of research and development companies for developing in vitro diagnostic reagent products according to one embodiment of the present disclosure.
FIG. 6 is a configuration diagram for exemplarily illustrating a configuration for providing data obtained from the device for managing research and development of in vitro diagnostic reagent products to a research and development company as reference data for research and development, according to one embodiment of the present disclosure.
FIG. 7 is an exemplary diagram for illustrating a configuration for separately storing data according to a standard process and data according to a non-standard process among data of research and development companies according to one embodiment of the present disclosure.
FIG. 8 is a flow chart for research and development and production of reagent products provided to research and development companies by the device for managing research and development of in vitro diagnostic reagent products according to one embodiment of the present disclosure.

### BEST MODE FOR IMPLEMENTING THE INVENTION

Advantages and features of the present invention, and methods for achieving them, will become clear with reference to the embodiments described below in detail in conjunction with the accompanying drawings. However, the present invention is not limited to the embodiments disclosed below, but may be implemented in various different forms, and only the present embodiments make the disclosure of the present invention complete, and common knowledge in the art to which the present invention belongs It is provided to fully inform the holder of the scope of the invention, and the present invention is only defined by the scope of the claims.

In the description of the present invention, if it is determined that a detailed description of a related known function or configuration may unnecessarily obscure the subject matter of the present invention, the detailed description will be omitted. In addition, terms to be described later are terms defined in consideration of functions in the present invention, which may vary according to the intention or custom of a user or operator. Therefore, the definition should be made based on the contents throughout this specification.

The term 'device for managing research and development' used herein may provide research and development processes through research and development management programs to research and development companies. In addition, production processes through production management programs may be provided to research and development companies. Although the term 'production' is omitted in the 'device for managing research and development management', the 'device for managing research and development' may include the production management program.

The term 'research and development company' used herein includes a company that researches and develops in vitro diagnostic reagent products through the device for managing research and development or produces in vitro diagnostic reagents for which research and development has been completed as products.

In one embodiment of the present disclosure, the 'research and development company' may include a company that performs the research and development and production of the in vitro diagnostic reagent products.

In one embodiment of the present disclosure, the 'research and development company' may include a company that performs only the research and development of the in vitro diagnostic reagent products.

In one embodiment of the present disclosure, the 'research and development company' may include a company that performs only the production of the in vitro diagnostic reagent products.

The term 'in vitro diagnostic reagent products' used herein includes molecular diagnostic reagent products.

The term 'in vitro diagnostic reagent' used herein includes a molecular diagnostic reagent or a target nucleic acid detection reagent.

The term 'in vitro diagnostic' used herein refers to a technology that rapidly diagnoses diseases outside the body using substances derived from the human body such as blood, excretions, body fluids, and saliva. The in vitro diagnostics play an important role in clinical decision-making and are becoming an essential and specialized element in patient treatment. Representative examples of the in vitro diagnostics may include blood sugar measurements, pregnancy tests, and COVID-19 tests, and equipment for performing the in vitro diagnostics are called in vitro diagnostics (IVD) devices. The in vitro diagnostic devices are medical devices used for testing using specimens such as tissue, blood, and urine collected from the human body for the purpose of diagnosing and prognosing diseases, assessing health conditions, assessing the effectiveness of disease treatment, and preventing diseases. The in vitro diagnostics devices also include in vitro diagnostic reagent products.

In vitro diagnostics devices may be classified as follows according to the testing method:
i) Immunochemistry
ii) Self-monitoring Blood Glucose (SMBG)
iii) Point of Care Testing (POCT)
iv) Molecular Diagnostics
v) Hematology
vi) Clinical Chemistry
vii) Hemostasis or Coagulation
viii) Tissue Diagnostics

According to one embodiment of the present disclosure, research and development companies 410, 420, and 430 may research and develop and produce various in vitro diagnostic reagent products, which are distinguished as described above.

According to one embodiment of the present disclosure, research and development companies 410, 420, and 430 may research and develop and produce molecular diagnostic reagent products for iv) a molecular diagnostic test method.

According to one embodiment of the present disclosure, a device 100 for managing research and development may provide at least one of the research and development companies 410, 420, and 430 with a research and development management program 121 used for research and development of in vitro diagnostic reagent products. A research and development process provided by the research and development management program 121 of the device 100 for managing research and development enables the research and development companies 410, 420, and 430 to develop the in vitro diagnostic reagent products using the same technology.

According to one embodiment of the present disclosure, the device 100 for managing research and development may provide at least one of the research and development companies 410, 420, and 430 with a production management program 122 used for manufacturing the in vitro diagnostic reagent products. A production process provided by the production management program 122 of the device 100 for managing research and development enables the research and development companies 410, 420, and 430 to manufacture the in vitro diagnostic reagent products using the same technology.

According to one embodiment of the present disclosure, the device 100 for managing research and development may provide at least one of the research and development companies 410, 420, and 430 with the research and development management program 121 used for research and development of the in vitro diagnostic reagent products and the production management program 122 used for manufacturing the in vitro diagnostic reagent products.

That is, the research and development companies 410, 420, and 430 may perform the research and development and/or production of the in vitro diagnostic reagent products using the research and development management program 121 and/or the production management program 122 provided by the device 100 for managing research and development.

The device 100 for managing research and development may obtain research and development data generated during the research and development process in response to the research and development of the in vitro diagnostic reagent products performed by the research and development companies 410, 420, and 430. The device 100 for managing research and development may store the obtained research and development data.

The device 100 for managing research and development may select necessary data from among the pieces of research and development data obtained from each research and development company 410, 420, and 430 to generate reference data for research and development.

According to one embodiment of the present disclosure, the device 100 for managing research and development may select necessary data from among the stored research and development data to generate the reference data for research and development, and selectively provide the generated reference data for research and development to the research and development companies 410, 420, and 430.

According to one embodiment of the present disclosure, the device 100 for managing research and development may select necessary data from among the stored research and development data, process the selected research and development data to generate the reference data for research and development, and selectively provide the generated reference data for research and development to the research and development companies 410, 420, and 430.

The device 100 for managing research and development may obtain production data generated during the production process in response to the production of the in vitro diagnostic reagent products performed by the research and development companies 410, 420, and 430. The device 100 for managing research and development may store the obtained production data.

The device 100 for managing research and development may select necessary data from among the production data obtained from each research and development company 410, 420, and 430 and generate reference data for research and development.

According to one embodiment of the present disclosure, the device 100 for managing research and development may select necessary data from among the stored production data to generate the reference data for research and development, and selectively provide the generated reference data for research and development to the research and development companies 410, 420, and 430.

According to one embodiment of the present disclosure, the device 100 for managing research and development may select necessary data from among the stored production data, process the selected production data to generate the reference data for research and development, and selectively provide the generated reference data for research and development to the research and development companies 410, 420, and 430.

According to one embodiment of the present disclosure, the research and development companies 410, 420, and 430 possess the same technology for research and development and/or production of the in vitro diagnostic reagent products. The above-described same technology may include research and development technology and/or production technology for the in vitro diagnostic reagent products.

According to one embodiment of the present disclosure, the research and development companies 410, 420, and 430 possess the same technology for research and development and/or production of the molecular diagnostic reagent products. The above-described same technology may include the research and development technology and/or production technology for the molecular diagnostic reagent products.

According to one embodiment of the present disclosure, the same technology or the same molecular diagnostic technology possessed by the research and development companies 410, 420, and 430 may be provided from the device 100 for managing research and development.

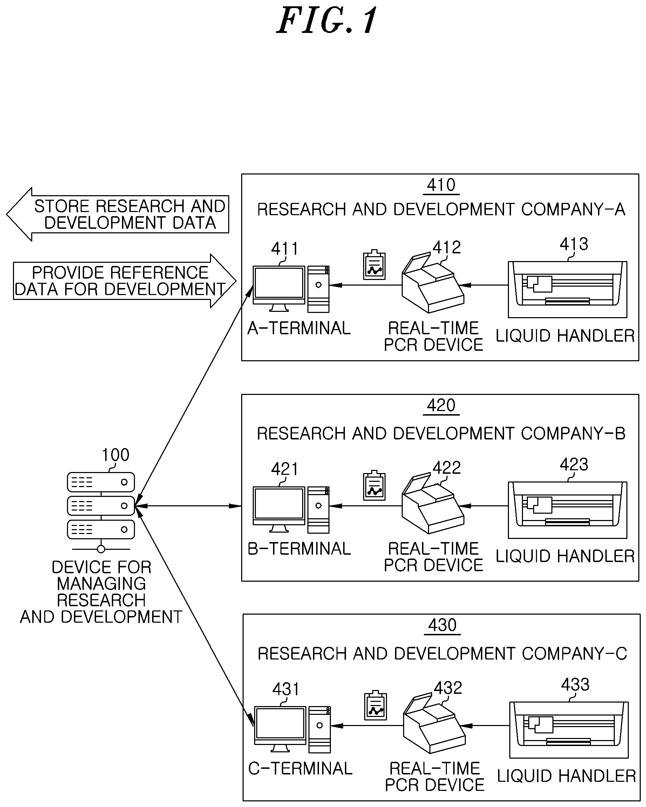

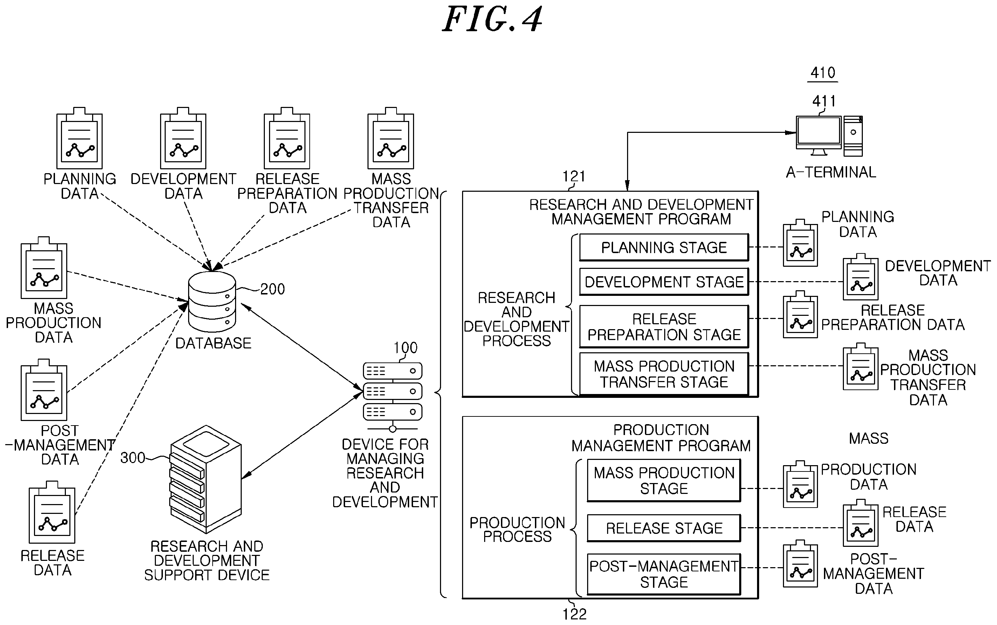

FIG. 1 is a configuration diagram illustrating an environment for developing in vitro diagnostic reagent products, including a device for managing research and development of in vitro diagnostic reagent products and research and development companies connected via a communication network, according to the present disclosure. FIG. 4 is a configuration diagram illustrating each stage of a process for developing in vitro diagnostic reagent products and data obtained through the process according to one embodiment of the present disclosure. As illustrated in FIGS. 1 and 4, the device 100 for managing research and development for the development of the in vitro diagnostic reagent products is connected to the plurality of research and development companies 410, 420, and 430 via a communication network. The device 100 for managing research and development provides the research and development program 121 such that the research and development companies 410, 420, and 430 may develop the in vitro diagnostic reagent products, respectively.

The device 100 for managing research and development that provides the research and development program 121 is a computing device composed of a terminal or a server. The plurality of terminals and servers may be provided in various forms.

The device 100 for managing research and development in FIG. 1 may provide the research and development management program 121, enabling the plurality of research and development companies 410, 420, and 430 to develop the in vitro diagnostic reagent products, independently.

The research and development management program 121 commonly provides the research and development companies 410, 420, and 430 with the same research and development process that allows the research and development of the in vitro diagnostic reagent products to be executed using the same technology. The same technology is technology required to develop the in vitro diagnostic reagent products, preferably the molecular diagnostic reagent products. The research and development companies 410, 420, and 430 may use the same technology required to develop the molecular diagnostic reagent products.

According to one embodiment of the present disclosure, the above-described same technology used for research and development of the molecular diagnostic reagent products includes at least one technology selected from the group consisting of a target gene sequence and amplicon determination technology, a candidate oligonucleotides design technology for a primer and a probe, an oligonucleotide structure technology, a signal generation mechanism indicating presence of a target, a technology for differentiating multiple signals generated from one label in one channel, a signal processing technology, a positive/negative determination technology, a nucleic acid extraction technology, non-clinical and clinical trial protocols, a clinical know-how by country, a document method for regulatory approval, and a raw material quality control (QC) know-how.

The above-described same technology only describes representative technologies used in the research and development process of the molecular diagnostic reagent products, and does not preclude the use of various other same technologies

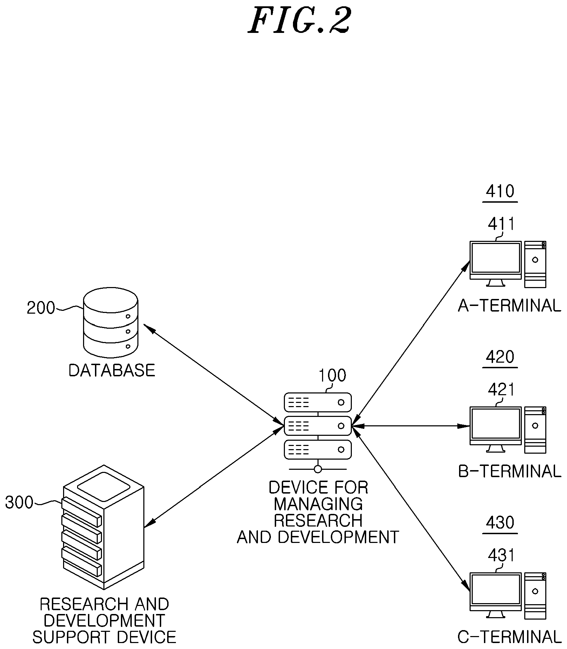

The research and development management program 121 stores, in a database 200, the research and development data of each research and development company 410, 420, and 430 obtained while using the research and development equipment for research and development of the in vitro diagnostic reagent products according to the above-described same research and development process. The database 200 may be described below in detail with reference to FIG. 2.

According to one embodiment of the present disclosure, the plurality of identical in vitro diagnostic reagent products researched and developed by the research and development companies 410, 420, and 430 using the same research and development process of the research and development management program 121 use the same raw materials.

In particular, the same raw materials may be used to research and develop the molecular diagnostic reagent products. The research and development data generated by the research and development using the same raw materials may have data compatibility such that the research and development data may be compared with each other.

According to one embodiment of the present disclosure, the same raw materials used to research and develop the molecular diagnostic reagent products may include oligonucleotides that specifically hybridize to target nucleic acids, enzymes, labels, auxiliary components, extraction reagents, and consumables.

According to one embodiment of the present disclosure, the oligonucleotide may include a primer and/or a probe.

According to one embodiment of the present disclosure, the enzyme may include nucleic acid polymerases, nucleic acid cleavage enzymes, and polymerases with cleavage enzyme activity.

According to one embodiment of the present disclosure, the other components may include buffers, metal ions, dNTPs, and salts.

According to one embodiment of the present disclosure, the extraction reagent may include lysis buffers, binding buffers, elution buffers, and magnetic beads.

According to one embodiment of the present disclosure, the consumables may include various types of plates and various types of tubes.

The above-described same raw materials only describe representative raw materials used in the research and development process of the molecular diagnostic reagent products, and do not preclude the use of various other raw materials.

The device 100 for managing research and development is connected to at least one of various terminals and equipment used by the research and development companies 410, 420, and 430 to develop the in vitro diagnostic reagent products via the communication network.

The research and development companies 410, 420, and 430 may include the company terminals 411, 421, and 431 connected to the device 100 for managing research and development via the communication network. The research and development companies 410, 420, and 430 may include a plurality of pieces of research and development equipment capable of researching and developing the in vitro diagnostic reagent products.

In one embodiment of the present disclosure, the company terminals 411, 421, and 431 may be computers, such as a personal computer (PC), a desktop computer, a laptop computer, a smartphone, and a tablet PC, or any devices including the computers. The computer is a device having one or more general purpose or special purpose processors, memory, storage, and network components (wired and/or wireless). The above-described devices may run an operating system, such as a Microsoft Windows-compatible operating system (OS), Apple OS X or iOS, a Linux distribution, or Google Android OS.

According to one embodiment of the present disclosure, the company terminals 411, 421, and 431 are an interface for interacting with the device 100 for managing research and development of the present disclosure, and may use web browsers, such as Microsoft Internet Explorer, Mozilla Firefox, Google Chrome, Apple Safari, and/or Opera. In another embodiment, the company terminals 411, 421, and 431 may execute a dedicated application for accessing the device 100 for managing research and development.

In one embodiment of the present disclosure, the research and development equipment may include a nucleic acid extraction device (not illustrated).

In one embodiment of the present disclosure, the research and development equipment may include liquid handlers 413, 423, and 433.

In one embodiment of the present disclosure, the research and development equipment may include real-time polymerase chain reaction (PCR) devices, 412, 422, and 432.

In one embodiment of the present disclosure, the research and development companies 410, 420, and 430 may further include various pieces of research and development equipment directly or auxiliary used to research and develop the in vitro diagnostic reagent products in addition to the nucleic acid extraction devices (not illustrated), the liquid handlers 413, 423, and 433, and the real-time polymerase chain reaction devices 412, 422, and 432 described above.

In one embodiment of the present disclosure, each piece of research and development equipment of the research and development companies 410, 420, and 430 is established with a communication connection to enable data transmission and reception with the company terminals 411, 421, and 431. Each of the company terminals 411, 421, and 431 may receive the research and development data output from the research and development equipment and provide the received research and development data to the device 100 for managing research and development. The company terminals 411, 421, and 431 may process each piece of research and development data and provide the processed research and development data to the device 100 for managing research and development.

In one embodiment of the present disclosure, the in vitro diagnostic reagent products are specifically the molecular diagnostic reagent products.

In FIG. 1, the research and development companies 410, 420, and 430 are companies that research and develop the molecular diagnostic reagent products. The research and development company-A 410 may include the company-A terminal 411, the real-time polymerase chain reaction device 412, and the liquid handler 413. The research and development company-B 420 may include the company-B terminal 411, the real-time polymerase chain reaction device 412, and the liquid handler 413. The research and development company-C 430 may include the company-C terminal 411, the real-time polymerase chain reaction device 412, and the liquid handler 413. That is, each research and development company 410, 420, and 430 connected to the device 100 for managing research and development may include the same company terminals 411, 421, and 431, the real-time polymerase chain reaction devices 412, 422, and 432, and the liquid handlers 413, 423, and 433, respectively. Although not illustrated in FIG. 1, the research and development companies 410, 420, and 430 may include a nucleic acid extraction device.

According to one embodiment of the present disclosure, the real-time polymerase chain reaction device 412 is a device including nucleic acid amplification and nucleic acid detection functions.

The above-described company terminals 411, 421, and 431, real-time polymerase chain reaction devices 412, 422, and 432, liquid handlers 413, 423, and 433, nucleic acid extraction devices (not illustrated), etc., may be included in the research and development equipment.

In one embodiment of the present disclosure, the research and development equipment is the same equipment, and the same equipment may be calibrated using the same calibration. The same calibration may be measured by the same calibration tools, and the calibration may be performed by the same standard value.

For example, the same research and development equipment may be measured using the same calibration tools, and when the measured result value falls within an error range presented by the same standard value, the measured research and development equipment may be continuously used. In addition, when the measured result value is out of the error range, the calibration by the same standard value may be performed, and the calibrated research and development equipment may be used.

The matters related to the above-described calibration are applicable only when the research and development equipment is the same equipment, and the same equipment is used under the calibration by the same standard value.

In another embodiment of the present disclosure, the research and development equipment is the same equipment, and the same equipment may operate by the same operation software. The operation software may be installed in the research and development equipment or a control terminal of the research and development equipment to operate a driving system that constitutes the research and development equipment. Therefore, the same operation software may control the performance of the driving system such that each pieces of the same research and development equipment operates under the same control commands.

In one embodiment of the present disclosure, the operation software of the research and development equipment includes a command for operating the real-time polymerase chain reaction device.

In one embodiment of the present disclosure, the operation software of the research and development equipment includes a command for operating the liquid handler.

In one embodiment of the present disclosure, the operation software of the research and development equipment includes a command for operating the nucleic acid extraction device.

The research and development data obtained from each of the research and development companies 410, 420, and 430 may be generated from the same operation software and/or the same analysis software for each of the same type of research and development equipment described above. Accordingly, the research and development data of each research and development company 410, 420, and 430 may have data compatibility such that the research and development data may be compared with one another.

According to another embodiment of the present disclosure, the same type of equipment among the pieces of research and development equipment may share the same manufacturer and model name or may be equivalently updated research and development equipment.

For example, in the above-described implementation example, when one research and development company has two liquid handlers, each liquid handler may be defined as the same type of research and development equipment. In addition, if two or more research and development companies each have one or more liquid handlers, each liquid handler may be defined as the same type of research and development equipment.

In order to provide uniform and consistent performance, the research and development equipment should be calibrated based on the same standard value. In particular, medical devices, etc., should be periodically calibrated in accordance with the National Standards Basic Act. For the calibration of such research and development equipment, the same calibration tools may be used based on the same standard value.

The research and development equipment may achieve the consistent performance and thus produce equivalent research and development results only when they are calibrated with the same standard value and operated by the same operation software. Therefore, each of the pieces of the same research and development equipment should be operated using the same operation software, with the same version of the operation software applied.

Source data output from the research and development equipment may be processed by analysis software to adjust result values, interpreted, provided to additional other software, or stored in a database.

The analysis software used for research and development of the molecular diagnostic reagent products may include at least one selected from the group consisting of a baselining algorithm, an amplification curve fitting algorithm, a Ct value determination algorithm, a melting curve analysis algorithm, and a positive/negative determination algorithm.

In the real-time PCR reaction, the baseline refers to a signal in an initial cycle of the PCR reaction. Typically, the baseline ranges from about 3 to 15 cycles. In this case, a change in a fluorescence signal does not appear significantly. The low signal of the baseline may be the background or noise of the amplification reaction. In the real-time PCR, the baseline may be set by automatically analyzing the user's analysis or amplification curve. Since the baseline setting affects the determination of an accurate Ct value, the baseline should be set carefully. In this case, a baselining algorithm automatically analyzes the amplification curve to set the baseline.

The amplification curve fitting algorithm described above is also called 'fitted curve' or 'curve fitting' and may generate a curve that best matches data points of the amplification data set. According to one embodiment of the present disclosure, the amplification curve fitting algorithm may be expressed as a mathematical function. According to one embodiment of the present disclosure, the amplification curve fitting algorithm may be implemented using a mathematical function selected from the group consisting of a polynomial function, an exponential function, a logarithmic function, a sigmoid function, a logistic function, and a Gompertz function.

The cycle threshold (Ct) value means a cycle number of a fluorescence signal indicating a contact point with the threshold. The cycle threshold refers to a value used to measure the amount of template. Theoretically, doubling the amount of template causes the Ct value to appear one cycle earlier. The above-described baseline is a weak signal that occurs at the beginning of the PCR, and the point at which the pattern of increase in the amplification signal is clearly distinguished is called the threshold. In this case, the Ct value determination algorithm is to obtain the Ct value, which is the cycle number that indicates the contact point with the threshold, and may be implemented in the analysis software.

Melting analysis means obtaining a signal indicating the presence of an extended duplex through melting of the extended duplex, and includes a method for measuring signals at two different temperatures, melting curve analysis, and melting pattern analysis. In the present disclosure, the algorithm for the melting curve analysis described above is included in the analysis software, but the algorithm for the melting pattern analysis may also be included in the analysis software, if necessary.

The melting curve or a hybridization curve may be obtained by the conventional techniques, such as those described in, for example, U.S. Patent Nos. 6,174,670 and 5,789,167, Drobyshev et al., Gene 188:45 (1997); Kochinsky and Mirzabekov, Human Mutation 19:343 (2002); Livehits et al., J. Biomol. Structure Dynam. 11:783 (1994); and Howell et al., Nature Biotechnology 17:87 (1999).

The positive/negative determination algorithm may process data obtained from the amplification reaction and perform the positive/negative reading of the target based on the Ct value and the pattern of increase in the fluorescence signal. A positive control substance used for the positive/negative reading should show positive results, and a negative control substance should show negative results. The positive/negative determination algorithm determines that the target is positive when the Ct value of the target is less than or equal to the determined Ct value. However, when the results of the positive and negative control substances are different from the expected results, the results may be determined as invalid regardless of the target results.

According to one embodiment of the present disclosure, the research and development companies 410, 420, and 430 may research and develop the in vitro diagnostic reagent products using the pieces of the above-described research and development equipment.

According to one embodiment of the present disclosure, the research and development companies 410, 420, and 430 may research and develop the molecular diagnostic reagent products using the pieces of the above-described research and development equipment.

The research and development management program 121 of the device 100 for managing research and development illustrated in FIG. 4 provides the research and development process for research and development of the in vitro diagnostic reagent products.

According to one embodiment of the present disclosure, the research and development process of the research and development management program 121 is a standardized process.

According to one embodiment of the present disclosure, the research and development companies 410, 420, and 430 may partially employ non-standard research and development processes while using the research and development management program 121.

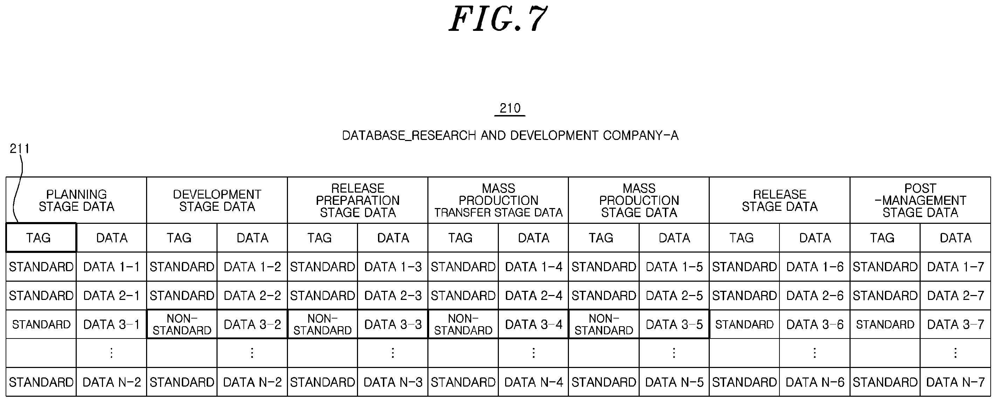

FIG. 7 is an exemplary diagram for illustrating a configuration for separately storing data according to a standard process and data according to a non-standard process among data of research and development companies according to one embodiment of the present disclosure. Referring to FIG. 7, the non-standard research and development process may mean changing and using at least one selected from the group consisting of the same research and development process, the same technology, the same equipment, the same raw materials, the same research and development equipment, the same operation software, and the same analysis software provided by the research and development management program 121.

The items included in the above-described non-standard research and development process are only representative items that may be provided or used by the research and development companies 410, 420, and 430 while using the research and development management program 121 for research and development of the in vitro diagnostic reagent products, and it does not exclude that other items are not included in the non-standard research and development process.

According to one embodiment of the present disclosure, the above-described non-standard research and development process may include at least one stage selected from the group consisting of a planning stage, a research and development stage, a release preparation stage, and a mass production transfer stage of the in vitro diagnostic reagent products.

The research and development data may also be generated in a stage selected as the non-standard research and development process among the above-described multiple stages, which may be referred to as the non-standard research and development data. The device 100 for managing research and development may obtain the non-standard research and development data generated through the non-standard research and development process, and may store the obtained non-standard research and development data in the database 210.

The device 100 for managing research and development may store the obtained non-standard research and development data in the database 210 such that the obtained non-standard research and development data may be identified from the standard research and development data.

According to one embodiment of the present disclosure, the research and development management program 121 of the device 100 for managing research and development may obtain the non-standard research and development data generated at any stage corresponding to the above-described non-standard research and development process and store the obtained non-standard research and development data in the database 210.

According to one embodiment of the present disclosure, a non-standard tag 211 may be assigned to the non-standard research and development data.

According to one embodiment of the present disclosure, the research and development management program 121 of the device 100 for managing research and development may obtain the non-standard research and development data to which the non-standard tag 211 is added and store the obtained non-standard research and development data in the database 210.

The research and development management program 121 of the device 100 for managing research and development may provide the reference data for research and development extracted at least partially based on the non-standard research and development data including the non-standard tag 211 stored in the database 210 to the research and development companies 410, 420, and 430.

The research and development companies 410, 420, and 430 may utilize the reference data for research and development based on the non-standard research and development data for research and development of the in vitro diagnostic reagent products.

According to one embodiment of the present disclosure, the device 100 for managing research and development may obtain a result in which a non-standard research and development process is determined to be a standardization target at least partially based on the above-described non-standard research and development data. Depending on the obtaining of the determination result, the device 100 for managing research and development may further include the non-standard research and development process in the above-described standardized research and development process.

For example, at least one of the research and development companies 410, 420, and 430 may research and develop specific in vitro diagnostic reagent products according to the standardized research and development process. In this case, in order to develop new in vitro diagnostic reagent products and/or new technologies, the research and development may be performed by separately applying the non-standard research and development process without using the existing standardized research and development process.

According to one embodiment of the present disclosure, the above-described non-standard research and development process may be applied to any one stage of the standardized research and development process and performed.

According to one embodiment of the present disclosure, the above-described non-standard research and development process may be applied to any two or more stages of the standardized research and development process and performed.

When the development of the in vitro diagnostic reagent products and/or new technology development are completed through the non-standard research and development process according to the above-described implementation example, a review may be performed to incorporate into the non-standard research and development process applied by the research and development company in the standardized research and development process.

According to one embodiment of the present disclosure, the review of the non-standard research and development process may be automatically conducted by AI in the device 100 for managing research and development.

According to one embodiment of the present disclosure, the review of the non-standard research and development process may be conducted based on an agreement of the research and development companies 410, 420, and 430.

According to one embodiment of the present disclosure, the review of the non-standard research and development process may be performed by a separate institution that operates the device 100 for managing research and development.

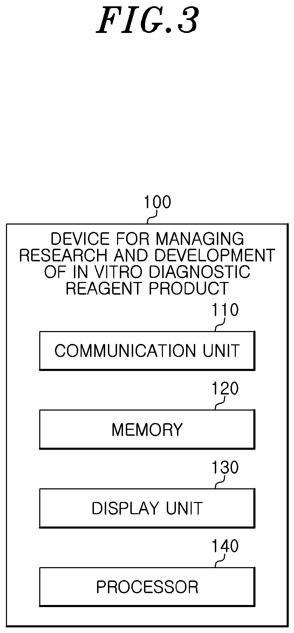

FIG. 3 is a block diagram exemplarily illustrating a configuration of the device for managing research and development of in vitro diagnostic reagent products according to the present disclosure. As illustrated in FIG. 3, the device 100 for managing research and development includes a communication unit 110, a memory 120, a display unit 130, and a processor 140. However, the configuration diagram illustrated in FIG. 3 is merely exemplary. For example, the device 100 for managing research and development may be implemented so as not to include the display unit 130 or at least one of the components illustrated in FIG. 3, or may be implemented so as to include components not illustrated in FIG. 3.

First, the communication unit 110 may be configured regardless of a communication mode, such as wire and wireless, and may be configured with various communication networks, such as a short-range communication network (personal area network (PAN)) and a local area network (wide area network (WAN)). In addition, the communication unit 110 may operate based on the known World Wide Web (WWW). Alternatively, a wireless transmission technology used for short-distance communication, such as infrared data association (IrDA) or Bluetooth, may be used.

According to one embodiment, the communication unit 110 may be used to receive or input the above-described action information or review criteria from an external computer, etc.

The memory 120 may mean <0065> any type of storage medium. For example, the memory 120 may include at least one type of storage medium selected from the group consisting of a flash memory type storage medium, a hard disk type storage medium, a multimedia card micro type storage medium, a card type memory (e.g., an SD or XD memory, or the like), a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disk.

In addition, the memory 120 may be a storage medium or a computer-readable recording medium that stores computer software that causes the processor 140 to perform operations according to embodiments of the present disclosure. At least one command that may be executed by the processor 140 may be stored in the memory 120. In addition, at least one of these commands may constitute software.

In this case, the software may be a pre-trained model implemented and operated by artificial intelligence (AI). For example, a model implemented or trained by machine learning or deep learning to perform a predetermined function may be stored in the memory 120.

Of course, a software module that is not implemented by the artificial intelligence but implemented to be operated in a general manner, for example, rule-based, may also be stored in this memory 120.

Meanwhile, the memory 120 may store any form of information generated or determined by the processor 140 and any form of information received by the previous cycle management device 100 through the communication unit 110.

According to one embodiment of the present disclosure, the research and development management program 121 is implemented in the memory 120. The research and development management program 121 is provided to research and development companies that perform the research and development of the in vitro diagnostic reagent products among the research and development companies 410, 420, and 430.

According to one embodiment of the present disclosure, the production management program 121 is implemented in the memory 120. The production management program 122 is provided to research and development companies that perform the production of the in vitro diagnostic reagent products among the research and development companies 410, 420, and 430.

According to one embodiment of the present disclosure, any one of the research and development companies 410, 420, and 430 may perform the research and development and production of the in vitro diagnostic reagent products.

According to one embodiment of the present disclosure, at least one of the research and development companies 410, 420, and 430 may perform either research and development or production of the in vitro diagnostic reagent products.

According to one embodiment of the present disclosure, the database may be implemented in the memory 120. In this sense, FIGS. 2 and 4 illustrate that the research and development data is stored in the database 200 connected to the device 100 for managing research and development via the communication network. The storage medium for storing the research and development data may be specifically described with reference to FIG. 2.

FIG. 2 is a configuration diagram conceptually illustrating a device connected to the device for managing research and development of in vitro diagnostic reagent products according to one embodiment of the present disclosure. As illustrated in FIG. 2, the device 100 for managing research and development is connected to the research and development companies 410, 420, and 430 via the communication network. Preferably, the device 100 for managing research and development is connected to the company terminals 411, 421, and 431 possessed by each research and development company 410, 420, and 430 via the communication network.

The device 100 for managing research and development may obtain various pieces of research and development data generated by each research and development company 410, 420, and 430 while conducting the research and development of the in vitro diagnostic reagent products. In this case, the device 100 for managing research and development may be equipped with the database 200 for storing the obtained research and development data.

The configuration for storing the research and development data may use the memory 120 of FIG. 3 described above, but may use the database 200 configured separately as needed.

According to one embodiment of the present disclosure, the research and development data may be stored in the memory 120 of the device 100 for managing research and development.

According to one embodiment of the present disclosure, the research and development data may be stored in the database 200 connected to the device 100 for managing research and development via the communication network.

The database 200 has a storage space with the same structure allocated to each of the research and development companies 410, 420, and 430. The research and development companies 410, 420, and 430 perform the research and development using the same technology and the same research and development equipment through the same research and development process. As a result, the research and development data is generated, and the research and development data generated by each research and development company 410, 420, and 430 may have data compatibility such that they may be compared with each other.

The data compatibility means that the data types of the research and development data are identical to each other, and the data formats are structured to be identical to each other.

The research and development data generated at various stages of the research and development process may be in various forms.

According to one embodiment of the present disclosure, the data type is information about properties of data, and among text data, properties such as characters, strings, numbers, dates, and time may be given. The research and development data may be classified as follows according to the data type.
i) CHAR - Fixed length character data
ii) CHARACET - Fixed length character string information
iii) VARCHAR - Variable length character data
iv) NUMBER - Internal number Format, numeric data
v) DATE - Data about date and time

According to one embodiment of the present disclosure, the data format is the form in which data is configured, and may be classified into various forms of files. The research and development data may be classified as follows according to the data format.
i) Document file - TXT, DOC, XML, XLS, PPT, PDF, HWP, etc.
ii) Image file - JPG, BMP, PNG, TIFF, GIF, PSD, etc.
iii) Audio file - WAV, FLAC, TTA, MP3, MMF, AIFF, AAC, etc.
iv) Video file - MPEG-1, MPEG-2, MPEG-3, H.261, H.263, H.264, MKV, etc.
v) Other files - ZIP, EXE, CSV, HTML, etc.

That is, the database 200 is allocated with respective storage spaces for each of the research and development companies 410, 420, and 430, such that structured research and development data having data compatibility may be acquired and stored, thereby enabling mutual comparison among the respective research and development companies 410, 420, and 430.

According to one embodiment of the present disclosure, the database 200 may have the storage space with the same size provided to the research and development companies 410, 420, and 430.

According to one embodiment of the present disclosure, the database 200 may have the storage space with the same structure provided to the research and development companies 410, 420, and 430.

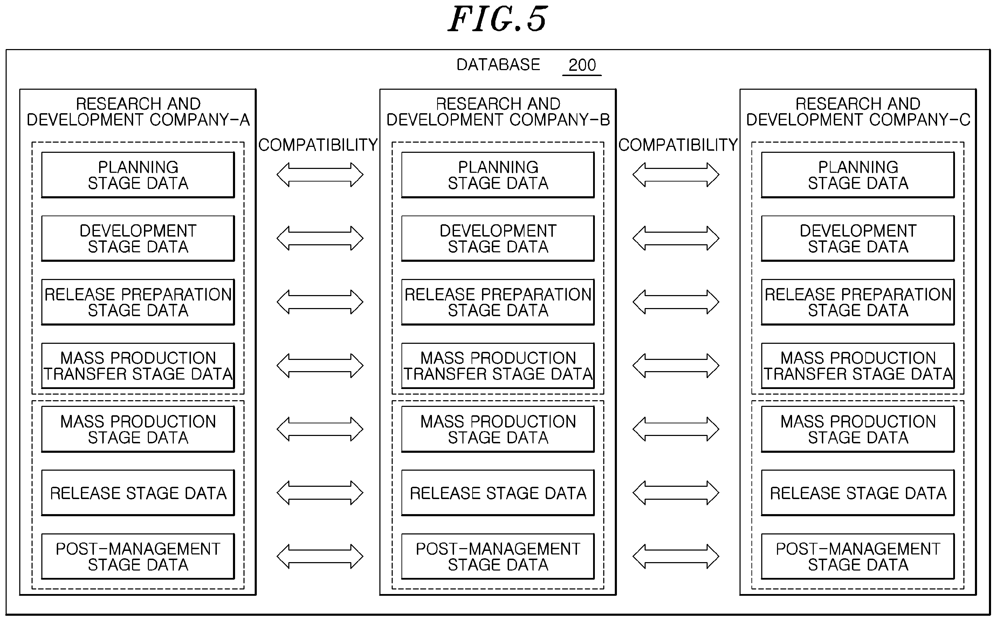

FIG. 5 is a block diagram exemplarily illustrating a concept of storing research and development data and production data of research and development companies for developing in vitro diagnostic reagent products according to one embodiment of the present disclosure. As illustrated in FIG. 5, the research and development data and/or the production data obtained according to research and development and/or production activities of the research and development companies 410, 420, and 430 may be stored in the database 200.

As described above, the database 200 may provide the storage space to the research and development companies 410, 420, and 430, and the research and development data and/or production data of each research and development company 410, 420, and 430 are stored in the corresponding storage space.

According to one embodiment of the present disclosure, the storage space of the database 200 allocated to each of the research and development companies 410, 420, and 430 is configured to separately store the research and development data of the research and development management program 121 and the production data of the production management program 122.

According to one embodiment of the present disclosure, the storage space of the database 200 allocated to each research and development company 410, 420, and 430 is configured to separately store the research and development data of the planning stage, the development stage, the release preparation stage, and the mass production transfer stage of the research and development management program 121.

According to one embodiment of the present disclosure, the storage space of the database 200 allocated to each of the research and development companies 410, 420, and 430 is configured to separately store the production data of the mass production stage, the release stage, and the post-management stage of the production management program 122.

According to one embodiment of the present disclosure, the research and development data or the production data stored in each of the research and development companies 410, 420, and 430 may be compatible with each other in the corresponding stages.

According to the above-described implementation examples of the present disclosure, the database 200 may store the research and development data of the in vitro diagnostic reagent products performed by the research and development companies 410, 420, and 430. The device 100 for managing research and development may update the research and development management program 121 and/or the production management program 122 using the research and development data.

According to one embodiment of the present disclosure, there may be the research and development companies 410, 420, and 430 performing research and development of a reagent for detecting a specific target nucleic acid in the in vitro diagnostic reagent products. The research and development companies 410, 420, and 430 may perform the research and development using the research and development process provided by the research and development management program 121 of the device 100 for managing research and development. In this case, the same problem may occur in the research and development data generated by the research and development companies 410, 420, and 430 at a specific stage. The device 100 for managing research and development may analyze the research and development data with the same problem and the research and development process of the stage for the research and development data to obtain a method for solving the same problem. The device 100 for managing research and development may update the research and development management program 121 based on the method for solving the same problem.

According to the above-described implementation example, the research and development process of the research and development management program 121 may be updated using the research and development data obtained from the research and development performance of the research and development companies 410, 420, and 430. Such an update may be performed as the research and development of the in vitro diagnostic reagent products of each of the research and development companies 410, 420, and 430 is performed by the standardized research and development process.

In another embodiment of the present disclosure, the device 100 for managing research and development may update the performance of the following items using at least one of the research and development data obtained from the research and development performance of the research and development companies 410, 420, and 430.
i) The research and development management program 121 may be updated using the research and development data. For example, updates such as the change (addition, modification, or deletion) in the screen interface of the research and development management program 121 due to the addition, modification, or deletion of the research and development data, and the change (addition, modification, or deletion) in functions used in the research and development management program 121 may be performed.
ii) The same technology used for research and development of the in vitro diagnostic reagent products may be updated using the research and development data. For example, the same technology used for research and development of the molecular diagnostic reagent products may include at least one technology selected from the group consisting of a target gene sequence and amplicon determination technology, a candidate oligonucleotides design technology for a primer and a probe, an oligonucleotide structure technology, a signal generation mechanism indicating presence of a target, a technology for differentiating multiple signals generated from one label in one channel, a signal processing technology, a positive/negative determination technology, a nucleic acid extraction technology, non-clinical and clinical trial protocols, a clinical know-how by country, a document method for regulatory approval, and a raw material QC know-how.
iii) The research and development process may be updated using the research and development data. For example, the research and development process may include the planning stage, the development stage, the release preparation stage, the mass production transfer stage, etc., but a third stage may be added or one or more of the above-described stages may be excluded as needed.
iv) Software used in the research and development equipment may be updated using the research and development data. The software used in the research and development equipment includes at least one of operation software for driving the research and development equipment or analysis software for analyzing the research and development data. For example, the research and development equipment includes the real-time polymerase chain reaction device, the liquid handler, the nucleic acid extraction device, etc., and the operation software for operating these devices may be updated. In addition, the analysis software used for research and development of the molecular diagnostic reagent products may include at least one selected from the group consisting of the baselining algorithm, the amplification curve fitting algorithm, the Ct value determination algorithm, the melting curve analysis algorithm, and the positive/negative determination algorithm.
v) The database 200 in which the research and development data is stored may be updated using the research and development data. For example, the update of the database 200 may include the changes (additions, modifications, or deletions) in the structure of the database 200, the size of the storage space allocated to each research and development company, the data compatibility for enabling the mutual comparison of the stored research and development data, etc.

According to one embodiment of the present disclosure, the research and development company-A 410 may perform the research and development of the reagent for detecting the specific target nucleic acid in the in vitro diagnostic reagent products. In this case, the research and development data is generated at a specific stage, and the device 100 for managing research and development may obtain the corresponding research and development data and store the obtained research and development data in the database 200. Thereafter, the research and development company-B 420 may also perform the research and development of the reagent for detecting the specific target nucleic acid described above. In response to the research and development of the reagent for detecting the specific target nucleic acid described above performed by the research and development company-B 420, the device 100 for managing research and development may provide the research and development data obtained from research and development company-A 410 to the research and development company-B 420 as the reference data for research and development. The research and development company-B 420 may perform the research and development of the reagent for detecting the specific target nucleic acid by using the provided reference data for research and development based on the research and development data of the research and development company-A 410.

As in the implementation example described above, the reference data for research and development may be generated based on the research and development data obtained by the research and development performed by research and development companies-A 410, 420, and 430 among the research and development companies 410, 420, and 430. The generated reference data for research and development may be provided to another research and development company-B 420. In other words, the use of the research and development data generated from different research and development companies as the reference data for research and development by a specific research and development company is made possible because the research and development of in vitro diagnostic reagent products by each research and development company 410, 420, and 430 is performed through the standardized research and development process.

Referring back to FIG. 2, the device 100 for managing research and development may include a research and development support device 300 that may support the research and development of the in vitro diagnostic reagent products of the research and development companies 410, 420, and 430.

According to one embodiment of the present disclosure, the research and development support device 300 may be provided in the device 100 for managing research and development. In this case, the research and development support device 300 may be referred to as a research and development support module.

The research and development companies 410, 420, and 430 may receive research and development support from the research and development support module implemented in the device 100 for managing research and development.

According to one embodiment of the present disclosure, the research and development support device 300 may be connected to the device for managing research and development via the communication network.

The research and development companies 410, 420, and 430 may receive research and development support from the research and development support device 300 connected to the device 100 for managing research and development.

According to one embodiment of the present disclosure, the research and development companies 410, 420, and 430 may access the research and development support device 300 through the device 100 for managing research and development.

According to one embodiment of the present disclosure, the research and development companies 410, 420, and 430 may directly access the research and development support device 300.

The research and development support device 300 may design candidate oligonucleotides required for research and development of a molecular diagnostic reagent product. The research and development of the molecular diagnostic reagent products may be performed using oligonucleotide that specifically hybridizes to target nucleic acids to be detected. In this case, the research and development support device 300 may generate a plurality of candidate oligonucleotides that specifically hybridize to the target nucleic acids and provide the generated candidate oligonucleotides to the research and development companies 410, 420, and 430.

Therefore, the research and development support device 300 or the research and development support module may include software to which at least one technology selected from the group consisting of the target gene sequence and amplicon determination technology capable of performing the design of the plurality of candidate oligonucleotides, and the candidate oligonucleotides design technology for a primer and/or a probe is applied.

According to one embodiment of the present disclosure, the research and development data may be generated from the terminals 411, 421, and 431 used by the research and development companies 410, 420, and 430 in the research and development.

According to one embodiment of the present disclosure, the research and development data may be generated from the research and development equipment 412, 413, 422, 423, 432, and 433 used by the research and development companies 410, 420, and 430 in the research and development.

According to one embodiment of the present disclosure, the research and development data may be generated in the research and development management program 121 using raw data generated from the terminals 411, 421, and 431 and/or the pieces of research and development equipment used by the research and development companies 410, 420, and 430 in the research and development.

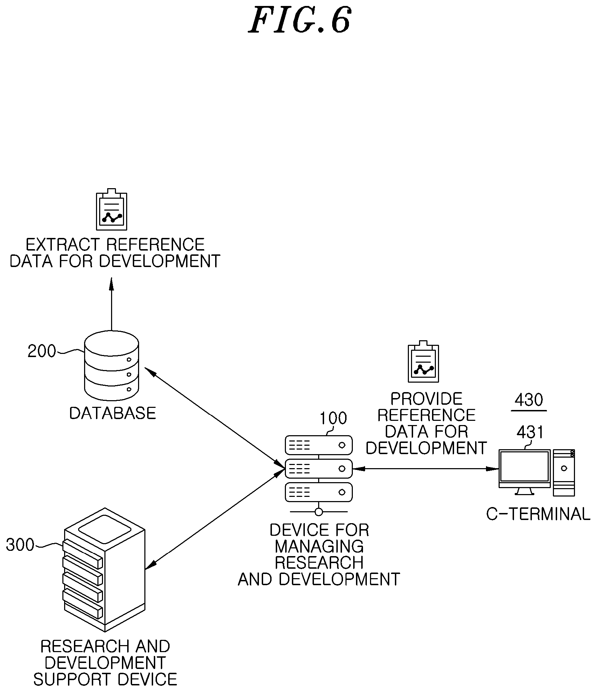

FIG. 6 is a configuration diagram for exemplarily illustrating a configuration for providing data obtained from the device for managing research and development of in vitro diagnostic reagent products to a research and development company as reference data for research and development, according to one embodiment of the present disclosure. As illustrated in FIG. 6, the database 200 provides the storage space to the research and development companies 410, 420, and 430.

According to one embodiment of the present disclosure, the database 200 is divided into separate storage spaces for storing the research and development data and the production data obtained from the research and development management program 121 and the production management program 122 provided by the device 100 for managing research and development, respectively.

According to one embodiment of the present disclosure, the database 200 is divided into separate storage spaces for storing research and development data according to multiple stages of each research and development process.

According to another embodiment of the present disclosure, the database 200 may store the research and development data in a manner that distinguishes multiple stages in the research and development process. That is, the database 200 is not divided into separate storage spaces according to each stage of the research and development process; however, the obtained research and development data may be added and stored with a label that may determine at which stage the obtained research and development data is generated. The above-described label may be tag data added to the research and development data.

According to one embodiment of the present disclosure, the database 200 is divided into separate storage spaces for storing production data according to multiple stages of each production process.

According to another embodiment of the present disclosure, the database 200 may store the production data such that multiple stages according to the production process are separated. That is, the database 200 is not divided into separate storage spaces according to each stage of the production process; however, the obtained production data may be added and stored with a label that may determine at which stage the obtained production is generated. The above-described label may be tag data added to the production data.

The research and development data generated and stored at each stage of the research and development process for research and development of the in vitro diagnostic reagent products by the research and development companies 410, 420, and 430 may be compatible with each other to allow comparison.

According to one embodiment of the present disclosure, the research and development management program 121 may provide the reference data for research and development extracted at least partially based on the research and development data stored in the database 200 to the research and development companies 410, 420, and 430.

That is, the data is compatible and comparable with each other, that is, the data compatibility means that when the above-described reference data for research and development is provided to at least one of the research and development companies 410, 420, and 430, at least one of the research and development companies may confirm the corresponding data using the research and development equipment that the research and development companies possess.

According to one embodiment of the present disclosure, the research and development data and the reference data for research and development using the same have data compatibility such that the corresponding data may be confirmed even if the research and development data and the reference data for research and development are provided to any research and development company.

According to one embodiment of the present disclosure, the research and development data and the reference data for research and development using the same have the data compatibility such that the data can be mutually compared with the research and development data generated by the research and development companies even if the research and development data and the reference data for research and development are provided to any research and development company.

In this case, one or more of the research and development companies 410, 420, and 430 may mutually compare the research and development data of the research and development that the research and development companies 410, 420, and 430 have directly performed with the research and development data that other research and development companies have performed.

According to one embodiment of the present disclosure, the research and development company may review the research and development performed through this comparison and then perform better research and development.

According to one embodiment of the present disclosure, the research and development company may use this comparison as data for verifying the research and development feasibility before performing the research and development of the in vitro diagnostic reagent products. The above-described verification of the research and development feasibility may be performed at the planning stage of the research and development process.

The device 100 for managing research and development may provide the research and development management program 121 including the same research and development process that may guide directing and strategy for research and development of reagent products to the research and development companies 410, 420, and 430 that research and develop the above-described reagent products.

The research and development management program may provide at least one selected from the group consisting of the same technology, the research and development equipment, the software, the algorithms, the research and development planning, the candidate oligonucleotides design, the clinical samples, the consumables, the enzymes, the nucleic acid extraction methods, the extraction reagents, and the nucleic acid amplification protocols, or the information corresponding thereto.

According to one embodiment of the present disclosure, the research and development process included in the research and development management program 121 may consist of the planning stage, the development stage, the release preparation stage, and the mass production transfer stage.

According to another embodiment of the present disclosure, the research and development process included in the research and development management program 121 may consist of at least one stage selected from the group consisting of the planning stage, the development stage, the release preparation stage, and the mass production transfer stage.

According to another embodiment of the present disclosure, the research and development process may be included in the research and development management program 121 by merging at least one stage selected from the group consisting of the planning stage, the development stage, the release preparation stage, and the mass production transfer stage, and may be included in the research and development management program 121.

In one embodiment of the present disclosure, the research and development process is a process for researching and developing the molecular diagnostic reagent products. Each stage included in the research and development process is described as follows.

### (i) Planning Stage

The planning stage is a proposal process for development of the molecular diagnostic reagent products. For example, in the planning stage, the development feasibility of a target nucleic acid detection reagent may be assessed. In addition, the verification of proposals, investment, and/or development plans, etc., for research and development may be performed. The planning stage includes plans for holding or securing clinical samples, plans for product design, etc.

The assessment of the development feasibility described above may be performed based on various factors. For example, the assessment of the development feasibility of the molecular diagnostic reagent products described above may include at least one selected from the group consisting of an assessment of the scope of application of the molecular diagnostic reagent products, that is, whether the molecular diagnostic reagent products are applicable to a plurality of fields, an assessment of whether the molecular diagnostic reagent products are applicable as multiple products in a single market, an assessment of whether there is demand in the market for the molecular diagnostic reagent products, an assessment of growth potential of the molecular diagnostic reagent products, that is, whether there is a niche market to which the molecular diagnostic reagent products are applicable, whether its potential growth is significant, etc., an assessment of commercialization of the molecular diagnostic reagent products, that is, whether the molecular diagnostic reagent products are applicable within a few years, etc., an assessment of market accessibility of the molecular diagnostic reagent products, that is, whether there are barriers to entry and the possibility of overcoming the barriers, or an assessment of the profitability of the molecular diagnostic reagent products, that is, whether the molecular diagnostic reagent products contribute to sales. The factors for the development feasibility assessment of the molecular diagnostic reagent product described above may be modified variously, and various factors for the development feasibility assessment are not excluded.

Depending on the results of the above-described development feasibility assessment, it may be determined whether to proceed to the next stage of the research and development process of the molecular diagnostic reagent product. For example, when the results of the preferable development feasibility assessment are calculated, the development stage may be performed during the research and development process. When the results of the development feasibility assessment are calculated to be unsuitable, the research and development process may be terminated.

According to one embodiment of the present disclosure, the research and development data generated in the above-described planning stage is stored in the database 200.

### (ii) Development Stage

The development stage includes designing the molecular diagnostic reagent, i.e., the target nucleic acid detection reagent. The target nucleic acid detection reagent that is researched and developed using the device 100 for managing research and development typically includes at least an oligonucleotide specific to the target nucleic acids. In addition, the target nucleic acid detection reagent may include an additional oligonucleotide that is not specific to the target nucleic acids. The oligonucleotide included in the above-described target nucleic acid detection reagent may act as a primer and/or a probe. In addition, the target nucleic acid detection reagent may additionally include at least one of the following components: labels, DNA polymerases, reverse transcriptase, dNTPs, Mg ions, potassium acetate (KCL), or buffers. Among the above components, components other than the oligonucleotide are non-specific (irrespective of the kind of target nucleic acids) to the target nucleic acids, whereas the oligonucleotide is designed to be specific (e.g., to be hybridized to the target nucleic acids) to the target nucleic acids. Therefore, the research and development of the target nucleic acid detection reagent is largely influenced by the performance of the oligonucleotide.

According to one embodiment of the present disclosure, the design of the target nucleic acid detection reagent includes designing the oligonucleotide included in the target nucleic acid detection reagent. Additionally, the design of the target nucleic acid detection reagent includes designing the oligonucleotide included in the target nucleic acid detection reagent and designing other components except for the oligonucleotide included in the target nucleic acid detection reagent.

According to one embodiment of the present disclosure, the design of the oligonucleotide included in the target nucleic acid detection reagent may be performed by the design method known in the art.

According to one embodiment of the present disclosure, the above-described design of the oligonucleotide may be performed using software that automatically designs a set of candidate oligonucleotide sequences that may be used to detect the target nucleic acids.

The development stage includes a process of performing the non-clinical trial of the molecular diagnostic reagent, i.e., the target nucleic acid detection reagent, during the research and development process.

The term 'non-clinical trial' used herein refers to a process of confirming the performance of the designed target nucleic acid detection reagent using a non-clinical sample. The term 'non-clinical sample' used herein refers to an artificial sample that contains a standard material of the target nucleic acid or a material such as a plasmid containing the target nucleic acid, rather than a sample obtained directly from a subject such as a human having or not having the target nucleic acid.

According to one embodiment of the present disclosure, the non-clinical trial of the target nucleic acid detection reagent may be performed by a performance test tool for the oligonucleotide included in the target nucleic acid detection reagent, and the performance test tool includes one or more of software, a device, and instructional information.

The performance test tool for the oligonucleotide refers to a tool that guides, instructs, assists, or facilitates the performance testing of the oligonucleotide.

According to one embodiment of the present disclosure, the non-clinical trial of the target nucleic acid detection reagent described above includes confirming the sensitivity and/or specificity of the oligonucleotide. More specifically, the non-clinical trial of the target nucleic acid detection reagent includes checking the sensitivity and/or specificity of oligonucleotides, and/or interference between oligonucleotides (e.g., dimer formation between oligonucleotides).

According to one embodiment of the present disclosure, the instruction information for the non-clinical trial of the target nucleic acid detection reagent described above includes a description of the experimental process and conditions of the performance testing of the oligonucleotides. The term 'instruction information' used herein is used interchangeably with protocol, manual, or tutorial for the experiment. In particular, the instruction information may include information about the sequential process of the experiment, the amount of the component used, the conditions of the reaction, etc. The instruction information may be in the form of a file such as a document, image, video, or audio that can be visually/audibly confirmed through the developer terminal and/or the development device, but is not limited thereto.

The development stage includes a process of performing the clinical trial of the molecular diagnostic reagent, i.e., the target nucleic acid detection reagent, during the research and development process.

The term 'clinical trial' used herein refers to the process of confirming whether the target nucleic acid detection reagent, whose performance has been confirmed through the non-clinical trial, is applicable to samples directly obtained from subjects. In other words, the clinical trial herein refers to a process of confirming whether the target nucleic acid detection reagent may accurately determine the presence or absence of the target nucleic acids in the sample obtained directly from subjects such as a human.

According to one embodiment of the present disclosure, the non-clinical sample is a sample artificially prepared to contain or not contain the target nucleic acid and generally does not contain any other components other than the target nucleic acids. In contrast, unlike the non-clinical sample, the clinical sample is a sample obtained from an actual subject and contains other components derived from the human body other than the target nucleic acids, which can be maintained even after the nucleic acid extraction. For this reason, even if the target nucleic acid detection reagent exhibits excellent performance in the target nucleic acid detection in the non-clinical sample, it may not be determined that the target nucleic acid detection reagent will exhibit the same performance in the clinical sample. Therefore, the clinical trial performed in the development stage confirms whether the target nucleic acid detection reagent that exhibited excellent performance in the non-clinical trial exhibits the same performance even in the clinical sample, and establishes experimental conditions to ensure that the target nucleic acid detection reagent exhibits the same or better performance in the clinical sample.

Since the clinical trial of the target nucleic acid detection reagent of this disclosure is similar to the non-clinical trial of the target nucleic acid detection reagent in terms of the used device, software, and instruction information, etc., a detailed description thereof will be omitted.

According to one embodiment of the present disclosure, the research and development data generated in the above-described development stage is stored in the database 200.

### (iii) Release Preparation Stage.

The release preparation stage includes a process of obtaining regulatory approval for sales of the molecular diagnostic reagent, i.e., the target nucleic acid detection reagent, during the research and development process.

The target nucleic acid detection reagent generally belongs to an in vitro diagnostic medical device, and should obtain regulatory approval according to the laws of each country to be sold. The regulatory approval differs from country to country. The regulatory approval generally involves technical documents on the purpose of use, operating principle, raw materials, performance, etc., and obtaining authorization based on this document.

The purpose of use included in the technical documents for the above-described regulatory approval refers to an inspection target of a product, a specimen type, a test item, a measurement principle, and a result determination method (quantification, qualification, etc.). In addition, the operating principle included in the technical documents for the regulatory approval refers to the physical, chemical, electrical, and mechanical principles applied during the reaction or measurement to achieve the purpose of use during product development. In addition, the raw materials included in the technical documents for the regulatory approval refer to main components that directly affect the reaction among products. In addition, the performance included in the technical documents for the regulatory approval encompasses a comparison of performance items equivalent to those of the product being compared. Specifically, the data for performance included in the technical document for the regulatory approval includes analytical performance test data, clinical performance test data, quality control test certificates for the finished product, data on standard materials used in analytical performance test, reference data for establishing conditions for specimen storage and handling, etc. In the present disclosure, the analytical performance test data includes data on analytical sensitivity, analytical specificity, precision, accuracy, etc., and the clinical performance test data is data tested on human-derived specimens to verify the performance and effectiveness of the target nucleic acid detection reagent, and includes clinical sensitivity, clinical specificity, etc.

According to one embodiment of the present disclosure, the research and development data generated in the above-described release preparation stage is stored in the database 200.

### (iv) Mass Production Transfer Stage

The mass production transfer stage includes a process of performing the mass production review based on the contents performed in the planning stage, the development stage, and the release preparation stage. The mass production may be approved when the mass production review is passed. The documents required in the mass production transfer stage may include at least one selected from the group consisting of a result report for each review of the molecular diagnostic reagent product, an implementation feasibility review report, a marketability review report, a specification document for the molecular diagnostic reagent product, a research note generated in a feasibility process, a manual document for performance assessment, a performance assessment result report, design verification data, a document for confirming validity of performance, a release plan, a transfer plan, a manual for transfer, a release checklist, a document for regulatory approval, and a user manual.

The above-described required documents are documents generated in the planning stage, the development stage, and the release preparation stage, respectively, and each of the required documents may be stored in the database 200 in each stage.

According to one embodiment of the present disclosure, the research and development data generated in the above-described mass production transfer stage is stored in the database 200.

The in vitro diagnostic reagent products being researched and developed by the above-described research and development management program are preferably molecular diagnostic reagent products. The research and development data obtained in the research and development of the molecular diagnostic reagent products is at least one selected from the group consisting of base sequence data of a specific target, candidate oligonucleotides design data used for a nucleic acid amplification reaction of a specific target, amplification curve data obtained from a nucleic acid amplification reaction experiment of a specific target, amplification curve slope data, Ct value data, end relative fluorescence unit (RFU) data, end RFU reading data, amplification efficiency data, exclusivity and inclusivity data of sensitivity and specificity, and setting values and positive/negative determination data of analysis software.

The kind of research and development data described above only selects and describes representative data that may be obtained from the development of the molecular diagnostic reagent, but do not exclude that various research and development data may be generated and obtained.

According to one embodiment of the present disclosure, the research and development data may include a traceable data log. The data log includes at least one piece of information selected from the group consisting of date, time, generated device, user, and change contents corresponding to the generation, viewing, output, modification, and/or deletion of the research and development data.

In the present disclosure, the research and development data obtained from the research and development companies 410, 420, and 430 have data compatiblility that allows mutual comparison. Therefore, in order to compare each research and development data, a data log in which a history of data can be confirmed is required, and the database 200 should be able to additionally store a data log for each research and development data.

When the research and development companies 410, 420, and 430 perform research and development for the development of the in vitro diagnostic reagent, these research and development companies 410, 420, and 430 may discover various problems at each stage of the research and development process. In this case, the research and development companies 410, 420, and 430 may generate trouble data, which are problems that occur. The device 100 for managing research and development obtains such trouble data and stores the obtained trouble data in the database 200.

In one embodiment of the present disclosure, the trouble shooting data corresponding to the above-described trouble data may be generated from the research and development company that generates the trouble data. That is, any one of the research and development companies that generate the trouble data may generate the trouble shooting data by solving a problem of the corresponding trouble data. According, any of the above-described research and development companies may each generate the trouble data and the corresponding trouble shooting data, respectively.

According to one embodiment of the present disclosure, the trouble shooting data corresponding to the above-described trouble data may be generated from another research and development company. Therefore, when one of the above-described research and development companies generates the trouble data, the corresponding trouble data may be provided to other research and development companies. One or more of other research and development companies may be able to solve the issues found in the corresponding trouble data, thereby generating the trouble shooting data. Therefore, any of the above-described research and development companies may generate the trouble data, and other research and development companies may generate the trouble shooting data corresponding to the above-described trouble data.

The device 100 for managing research and development may obtain the trouble data and/or the trouble shooting data generated in the embodiment and store the obtained trouble data in the database 200.

Here, the trouble means finding a problem, and the trouble shooting means the work of identifying and solving the cause when a problem occurs. Therefore, the trouble data for a problem that occurred during the research and development may be generated, and the trouble shooting data corresponding to the trouble may be generated to solve the problem.

According to one embodiment of the present disclosure, the trouble shooting may be performed by the following process.
i) Problem (trouble) definition - Confirm the problematic operation or cause
ii) Fact collection - Collect information about the corresponding problem, collect the method for reproducing the problem
iii) Cause inference - Infer the cause through the confirmed facts
iv) Determining investigation method - Deciding in what order to solve the problem based on symptoms and inferred cause, first investigating the most likely cause in general
v) Implementing investigation method - Investigating the problem based on the previously determined contents
vi) Observing results - Implementing the investigation method and recording the results, when the problem is not solved, returning to the iv) process
vii) Arranging and documenting the order in which the problem was solved.

Therefore, the trouble shooting data includes the contents documented in the vii) process.

The research and development companies 410, 420, and 430 may perform research and development for developing the in vitro diagnostic reagent products, and may also produce the developed in vitro diagnostic reagent products.

In one embodiment of the present disclosure, the research and development companies 410, 420, and 430 may produce the in vitro diagnostic reagent products using their respective production equipment.

To this end, the device 100 for managing research and development may provide the production management program 122 to the research and development company producing the in vitro diagnostic reagent products.

The research and development management program 121 and the production management program 122 may be selectively provided to each research and development company among the research and development companies 410, 420, and 430 depending on whether the company is performing a research and development project and a production project of the in vitro diagnostic reagent products.

Referring to FIG. 4, the device 100 for managing research and development includes the research and development management program 121 and the production management program 122.

In this case, the production management program 122 may provide the same production process capable of producing the in vitro diagnostic reagent products to the research and development companies. As described above, the production management program 122 is provided only to the research and development companies that produce the in vitro diagnostic reagent products among the research and development companies 410, 420, and 430. The device 100 for managing research and development may store the production data obtained while using the production equipment for producing the in vitro diagnostic reagent products in the database 200. The production data provided by the research and development companies 410, 420, and 430 has the data compatibility such that they can be compared with each other.

The data compatibility means that the data types of the production data are identical to each other, and the data formats are structured to be identical to each other. The contents related to the data compatibility may refer to the description of the above-described research and development data.

The production management program 122 may provide the reference data for research and development extracted at least partially based on the production data stored in the database 200 to the research and development companies 410, 420, and 430. The reference data for research and development may refer to the description of the above-described research and development data.

According to one embodiment of the present disclosure, the production data generated through the production process of the production management program 122 may include at least one selected from the group consisting of usage data of raw materials, inventory data of the raw materials, inbound/outbound data of the raw materials, trouble data generated during production, trouble shooting data, manufacturing number data, production schedule data by item of the in vitro diagnostic reagent products, production volume data, usage data of consumables for producing the in vitro diagnostic reagent products, inventory data of the consumables, inbound/outbound data of consumables, and voice of the consumer (VoC) data for producing the in vitro diagnostic reagent products.

According to one embodiment of the present disclosure, the production data generated through the production process managed by the production management program 122 may include quality control information (QC information) for quality control performed for at least one stage selected from the same production process. The quality control information is generated by each of the research and development companies 410, 420, and 430 receiving the production management program 122 using the same quality control tools.

According to one embodiment of the present disclosure, the production data may include raw material inventory information of the research and development companies 410, 420, and 430 producing the in vitro diagnostic reagent products through the same production process provided by the production management program 122 and/or product inventory information for the produced in vitro diagnostic reagent products.

The production management program 122 enables the research and development companies to supply raw materials at the appropriate time without causing disruptions in the production of the in vitro diagnostic reagent products by utilizing production schedule data and raw material inventory information.

The production management program 122 may check the product inventory information for the in vitro diagnostic reagent products when a supply request for any one of in vitro diagnostic reagent products manufactured by the research and development company occurs. In this way, when the product inventory for the in vitro diagnostic reagent products is insufficient, it is possible to request additional production of the in vitro diagnostic reagent products from the research and development company.

According to one embodiment of the present disclosure, the same production process provided by the production management program 122 may be divided into the mass production stage, the release stage, and the post-management stage. In this case, multiple stages provided in each process may be configured in various forms that may be combined into one stage as needed.

Each stage included in the production process provided by the production management program 122 is described as follows.

### (i) Mass Production Stage

The mass production stage includes the process of producing the molecular diagnostic reagent products, i.e., the target nucleic acid detection reagent products. The production of the target nucleic acid detection reagent refers to manufacturing the target nucleic acid detection reagent that has obtained regulatory approval for sale as described above. The production of the target detection reagent includes the following processes of:
a) synthesizing oligonucleotides
b) preparing components included in the target nucleic acid detection reagent excluding the above-described oligonucleotides
c) mixing the above-described oligonucleotides and other components
d) putting the above-described mixture into a container in a certain amount and packaging the container

According to one embodiment of the present disclosure, the production data generated in the above-described production stage is stored in the database 200.

### (ii) Release Stage

The release stage includes the process of selling the molecular diagnostic reagent products, i.e., the target nucleic acid detection reagent products. The sale of the target nucleic acid detection reagent products in the release stage may be performed in a manner known in the art.

According to one embodiment of the present disclosure, the sale of the target nucleic acid detection reagent product is preferably performed according to the production process provided by the production management program 122 of the device 100 for managing research and development.

According to one embodiment of the present disclosure, the production data generated in the above-described release stage is stored in the database 200.

### (iii) Post-management Stage

The post-management stage includes the process of obtaining the post-management data generated after the sale of the molecular diagnostic reagent products, i.e., target nucleic acid detection reagent products. The post-management data may include the voice of the customer (VoC) information on the sold molecular diagnostic reagent products. That is, when a problem occurs in the test results for determining whether a patient is infected with a pathogen, data for solving the corresponding problem is obtained. The post-management stage may obtain the above-described data and derive a solution based on the obtained data. For example, the trouble data generated during the use of the sold target nucleic acid detection reagent products may be collected and the trouble shooting data corresponding to the trouble data may be generated.

In the post-management stage, the change impact assessment may be performed to review whether the sold target nucleic acid detection reagent products have been changed, whether renewal of regulatory approval is required due to the change, whether performance is changed due to the change, etc., based on the generated trouble shooting data. For example, in the change impact assessment performed in the post-management stage, the following results may be derived: continuing to sell products without changing/modifying the products, performing a change in products and renewal of regulatory approval in response to the change, and stopping the sale of products, etc.

The results derived from the above-described change impact assessment are merely illustrative examples, and do not exclude the possibility that other results may be derived.

According to one embodiment of the present disclosure, the production data generated in the above-described post-management stage is stored in the database 200.

In one embodiment of the present disclosure, for the molecular diagnostic test, one or more products selected from the group consisting of a test reagent, an extraction reagent, oligonucleotide, and an enzyme may be manufactured.

The production equipment for manufacturing reagent products for a molecular diagnostic test may include at least one production module selected from the group consisting of a preprocessing module for a product container that receives products, a liquid handler that injects a reagent into the product container, a capping module or decapping module that closes or opens a cap on the product container, a vision inspection module for inspection during the production process of the reagent product, a labeling module, a packaging module, etc.

According to one embodiment of the present disclosure, among the pieces of production equipment, the same kind of equipment may be calibrated using the same standard value.

According to one embodiment of the present disclosure, the same kind of equipment among the pieces of production equipment may be operated by the same operation software.

According to one embodiment of the present disclosure, the same kind of equipment among the pieces of production equipment may be released with the same manufacturer and model name or may be equivalently updated production equipment.

For example, in the above-described implementation example, when one research and development company has preprocessing modules for two product containers, the preprocessing modules for each product container may be defined as the same kind of production equipment. In addition, when two or more research and development companies each have preprocessing modules for one or more product containers, the preprocessing modules for each product container may be defined as the same kind of production equipment.

In one embodiment of the present disclosure, the production equipment is the same type of equipment, and the same type of equipment may be applied with the same calibration. The same calibration may be measured by the same calibration tools, and the calibration may be performed by the same standard value.

For example, the same production equipment may be measured using the same calibration tools, and when the measured result value falls within an error range presented by the same standard value, the measured production equipment may be continuously used. In addition, when the measured result value is out of the error range, the calibration by the same standard value may be performed, and the calibrated production equipment may be used.

The above-described calibration is applicable when the production equipment is the same equipment and the same equipment is used through the calibration with the same standard value.

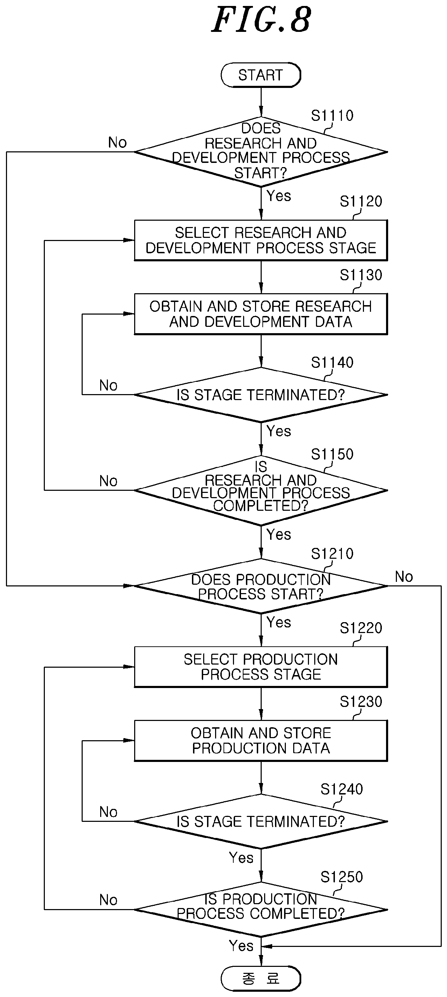

FIG. 8 is a flow chart for research and development and production of reagent products provided to research and development companies by the device for managing research and development of in vitro diagnostic reagent products according to one embodiment of the present disclosure. As illustrated in FIG. 8, the device 100 for managing research and development may provide the research and development management program 121 and the production management program 122 to the research and development companies 410, 420, and 430.

Among the research and development companies 410, 420, and 430, at least one research and development company may access the device 100 for managing research and development for research and development and/or production of the in vitro diagnostic reagent products. The research and development company described below with reference to FIG. 8 is described by being limited to research and development company-A 410 among the research and development companies 410, 420, and 430, but it does not exclude that the same may be applied to the remaining research and development companies 420 and 430.

The device 100 for managing research and development may receive a request for initiation of a research and development process provided by the research and development management program 121 from the accessed research and development company-A 410 (S1110).

According to one embodiment of the present disclosure, when the research and development company-A 410 does not request the initiation of the research and development process in step S1110, a request for initiation of the production process may be received (S1210).

In step S1110, when a request for initiation of the research and development process is made from the research and development company-A 410, the device 100 for managing research and development may receive a selection of the stage of the research and development process from the research and development company-A 410 (S1120).

The research and development company-A 410 may select the stage corresponding to the research and development stage from among multiple stages provided by the research and development management program 121 of the device 100 for managing research and development.

According to one embodiment of the present disclosure, multiple stages in the research and development process may include the planning stage, the development stage, the release preparation stage, and the mass production transfer stage.

According to one embodiment of the present disclosure, the research and development process in which any two or more of the respective stages of the above-described research and development process are combined may be provided to the research and development company-A 410.

According to one embodiment of the present disclosure, the research and development process in which one or more of the above-described respective stages is omitted may be provided to the research and development company-A 410.

The research and development management program 121 of the device 100 for managing research and development may obtain and store the research and development data corresponding to the stage selected by the research and development company-A 410 (S1130).

The implementation example described below may be performed before and/or after obtaining the research and development data of the step S1130.

According to one embodiment of the present disclosure, the research and development management program 121 may provide the research and development company-A 410 with the instruction information required at each stage of the research and development process. The instruction information required at the stage may include any one selected from the group consisting of a research and development plan sample, software, a list of clinical samples, a nucleic acid extraction method, a list of extraction reagents, a protocol for nucleic acid amplification, a candidate oligonucleotides design, an experimental process and conditions for a performance test on oligonucleotides, reference data for research and development, and a regulatory approval format for the in vitro diagnostic reagent products that are required for the research and development of the in vitro diagnostic reagent products by the research and development company-A 410.

The instruction information required in the above-described stage only describes the typical contents used in the research and development process of the in vitro diagnostic reagent products, and it is not excluded the possibility that various information may be further provided.

According to one embodiment of the present disclosure, the research and development management program 121 may approve the use of the research and development support device 300 to the research and development company-A 410. The approval of the use of the research and development support device 300 may be achieved by performing at least one selected from the group consisting of connection of a communication network such that the research and development company-A 410 may access the research and development support device 300, provision of an account (ID) and a password, provision of a uniform resource locator that can be accessed, and registration of an IP address of the research and development company-A terminal 411 that can be accessed.

The device 100 for managing research and development may further obtain and store the research and development data generated by the research and development company-A 410 while using the research and development support device 300. The research and development data generated while using the research and development support device 300 may be generated by the research and development support device 300 or by the research and development company-A 410.

The device 100 for managing research and development determines whether the selected stage of the research and development process has been terminated (S1140). The termination of the selected stage of the research and development process is performed by the research and development company-A 410.

According to one embodiment of the present disclosure, when the selected stage of the research and development process has been terminated, the research and development management program 121 of the device 100 for managing research and development may provide the next stage of the research and development process to the research and development company-A 410.

According to one embodiment of the present disclosure, even when the selected stage of the research and development process has been terminated, the addition, modification, and/or deletion, etc., of the research and development data may be performed at the request of the research and development company-A 410.

According to one embodiment of the present disclosure, even when the selected stage of the research and development process has been terminated, the above-described selected stage may be re-performed at the request of the research and development company-A 410.

In step S1140, when the stage that has been performed during the research and development process has been terminated, the research and development management program 121 receives a decision from the research and development company-A 410 whether to proceed the next stage (S1150). If the stage of the research and development process has not been terminated in step S1140, step S1130 may be continuously performed.

In step S1150, it may be confirmed whether the research and development process is terminated. First, in step S1140, the stage is terminated, and in step S1150, it may be confirmed that the research and development process is terminated. In this case, the research and development management program 121 may determine that the research and development process is terminated, and that the research and development of the in vitro diagnostic reagent products conducted by the research and development company-A 410 is completed.

When the research and development process has not been completed in step S1150, the research and development process is performed again from step S1120. In step S1120, the research and development management program 121 may select the stage of the research and development process from the research and development company-A 410.

The research and development management program 121 may repeatedly perform steps S1120 to S1150 until the research and development process is completed.

When it is confirmed that the research and development process is completed in step S1150, the device 110 for managing research and development may receive a request for the initiation of the production process provided by the production management program 122 from the accessed research and development company-A 410 (S1210).

According to one embodiment of the present disclosure, the research and development company-A 410 may be a company that performs only the research and development of the in vitro diagnostic reagent products. When the research and development company-A 410 is a company that performs only the research and development of the in vitro diagnostic reagent products, the device 100 for managing research and development may confirm that the production process is not initiated in step S1210.

According to one embodiment of the present disclosure, the research and development company-A 410 may be a company that performs both the research and development and production of the in vitro diagnostic reagent products. When the research and development company-A 410 is a company that performs the research and development and production of the in vitro diagnostic reagent products, the device 100 for managing research and development may receive a request for the initiation of the production process in step S1210. Alternatively, even if the research and development company-A 410 is a company that performs the research and development and production of the in vitro diagnostic reagent products, it may not perform the production process after the research and development process.

According to one embodiment of the present disclosure, the research and development company-A 410 may be a company that performs only the production of the in vitro diagnostic reagent products. When the research and development company-A 410 is a company that performs only the production of the in vitro diagnostic reagent products, the device 100 for managing research and development may confirm that the research and development process is not initiated in step S1110, and may receive a request for the initiation of the production process in step S1210.

The device 100 for managing research and development may receive from the research and development company-A 410 whether the above-described research and development process has been initiated and/or whether the production process has been initiated.

In step S1210, when it is confirmed that the research and development company-A 410 has initiated the production process, the device 100 for managing research and development may provide the research and development company-A 410 with the production management program 122. The research and development company-A 410 may perform the production process through the production management program 122.

According to one embodiment of the present disclosure, the production process provided by the production management program 122 may include the mass production stage, the release stage, and the post-management stage.

According to one embodiment of the present disclosure, the production process in which any two or more of the respective stages of the above-described production process are combined may be provided to the research and development company-A 410.

According to one embodiment of the present disclosure, the production process in which one or more of the respective stages of the above-described production process are omitted may be provided to the research and development company-A 410.

In step S1210, when a request for the initiation of the research and development process is made by the research and development company-A 410, the device 100 for managing research and development may receive the stage selection of the production process from the research and development company-A 410 (S1120). That is, the research and development company-A 410 may select the stage according to the production stage from among multiple stages provided by the production management program 122 of the device 100 for managing research and development.

The production management program 122 of the device 100 for managing research and development may obtain and store the production data corresponding to the stage selected by the research and development company-A 410 (S1230).

The embodiment described below may be performed before and/or after obtaining the production data in the step S1230.

According to one embodiment of the present disclosure, the production management program 122 may provide the research and development company-A 410 with the instructional information required at each stage of the production process.

The instruction information required at each stage may include any one selected from the group consisting of a list of raw materials, a standard quality control technique, a standard production schedule, a raw material ordering method, and a production equipment operation manual that are required for the production of the in vitro diagnostic reagent products by the research and development company-A 410.

The instruction information required in the above-described stage merely describes the typical contents used in the production process of the in vitro diagnostic reagent products, and does not exclude the possibility that various information may be further provided.

The device 100 for managing research and development determines whether the selected stage of the production process has been terminated (S1240). The termination of the selected stage of the production process is carried out by the research and development company-A 410.

According to one embodiment of the present disclosure, when the selected stage of the production process is terminated, the production management program 122 of the device 100 for managing research and development may either provide the next stage of the production process to the research and development company-A 410 or confirm whether the production process has been completed.

According to one embodiment of the present disclosure, even when the selected stage of the production process is terminated, the addition, modification, and/or deletion, etc., of the production data may be performed at the request of the research and development company-A 410.

According to one embodiment of the present disclosure, even after the selected stage of the production process is terminated, the above-described selected stage may be re-performed at the request of the research and development company-A 410.

In step S 1240, when the stage that has been performed during the production process is terminated, the production management program 122 determines whether the production process is completed from research and development company-A 410 (S1250). When the production process is not completed in step S1250, the production process may move to step S1220 and select a stage that has not been performed during the production process to continue to perform step S1230.

In step S1250, it may be confirmed whether the production process is terminated. First, in step S1240, the stage is terminated, and in step S1250, it may be confirmed that the production process is terminated. In this case, the production management program 122 may determine that the production process has been terminated, and that the production of the in vitro diagnostic reagent products conducted by the research and development company-A 410 has been completed.

Second, when the production process has not been completed in step S1250, it is performed again from step S1220. In step S1220, the production management program 122 may select the stage of the production process from the research and development company-A 410.

The production management program 122 may repeatedly perform steps S1220 to S1250 until the enitre production processes are completed.

According to one aspect of the present disclosure, there may be provided a computer-implemented method for research and development of in vitro diagnostic reagent products which is performed in a device for managing research and development of in vitro diagnostic reagent products using a memory, a processor, and one or more programs stored in the memory and configured to be executed by the processor, in which the device for managing research and development stores a research and development management program that is provided to research and development companies developing the in vitro diagnostic reagent products, the research and development management program includes: commonly providing the same research and development process to the research and development companies such that the research and development of the in vitro diagnostic reagent products is executed using the same technology; and storing the research and development data of each research and development company obtained while using the research and development equipment for the research and development of the in vitro diagnostic reagent products according to the same research and development process in a database, and the database is a database in which a storage space with the same structure is allocated to each of the research and development companies, and the research and development data of each of the research and development companies has data compatibility such that the research and development data is compared with each other.

Each component described in the above-described aspect of the present disclosure overlaps with the device for managing research and development of the in vitro diagnostic reagent products described with reference to FIGS. 1 to 8 described above, and thus, the description thereof is omitted.

According to one aspect of the present disclosure, there may be provided a computer-readable recording medium on which a computer program for managing research and development of in vitro diagnostic reagent products by a device for managing research and development of in vitro diagnostic reagent products is stored, in which the computer program performs: commonly providing the same research and development process to the research and development companies such that the research and development of the in vitro diagnostic reagent products is executed using the same technology; and storing the research and development data of each research and development company obtained while using the research and development equipment for the research and development of the in vitro diagnostic reagent products according to the same research and development process in a database, and the database is a database in which a storage space with the same structure is allocated to each of the research and development companies, and the research and development data of each of the research and development companies has data compatibility such that the research and development data is compared with each other.

Each component described in the above aspect of the present disclosure overlaps with the device for managing research and development of the in vitro diagnostic reagent products described with reference to FIGS. 1 to 8, and thus, the description thereof is omitted.

According to an aspect of the present disclosure, there may be provided a computer program stored on a computer-readable recording medium for allowing research and development companies to manage research and development of in vitro diagnostic reagent products by a device for managing research and development of in vitro diagnostic reagent products, in which the computer program performs: commonly providing the same research and development process to the research and development companies such that the research and development of the in vitro diagnostic reagent products is executed using the same technology; and storing the research and development data of each research and development company obtained while using the research and development equipment for the research and development of the in vitro diagnostic reagent products according to the same research and development process in a database, and the database is a database in which a storage space with the same structure is allocated to each of the research and development companies, and the research and development data of each of the research and development companies has data compatibility such that the research and development data is compared with each other.

Each component described in the above-described aspect of the present disclosure overlaps with the device for managing research and development of the in vitro diagnostic reagent products described with reference to FIGS. 1 to 8, and thus, the description thereof is omitted.

One or more of the embodiments described herein provide that the methods, technologies, and operations executed by the computing device are performed in a programmatic manner or as a computer-implemented method. The term "program" as used herein means using a code or a computer-executable command. Such command may be stored in one or more memory resources of the computing device. The steps performed in a programmatic manner may automatic or may not be automatic.

One or more of the embodiments described herein may be implemented using a device, equipment, or module. The device, equipment, or module may include a software component or hardware component that may perform a portion of the program or one or more of the mentioned tasks or functions. As used herein, the device or module may exist on a hardware component independently of another device or module. Alternatively, the device or module may be a shared element or process of another device, module, program, or apparatus.

The above description is merely illustrative of the technical idea of the present disclosure, and those skilled in the art to which the present disclosure pertains will be able to make various modifications and variations without departing from the essential quality of the present disclosure. Accordingly, the embodiments disclosed in the present disclosure are not intended to limit the technical idea of the present disclosure, but to explain it, and the scope of the technical idea of the present disclosure is not limited by these embodiments. The protection scope of the present invention should be interpreted by the following claims, and all technical ideas within the scope equivalent thereto should be interpreted as being included in the scope of the present invention.

### [Cross-Reference to Related Application]

The present application claims priority to Korean Patent Application No. 10-2022-0170746, filed on December 8, 2022, with the Korean Intellectual Property Office, the entire disclosure of which is incorporated herein by reference.

## Claims

1. A device for managing research and development of an in vitro diagnostic reagent product, comprising:
a memory that stores at least one instruction; and
a processor,
wherein the memory stores a research and development management program that is provided to research and development companies developing the in vitro diagnostic reagent product,
wherein the at least one instruction, when executed by the processor, causes the research and development management program to: commonly provide the same research and development process to the research and development companies such that the research and development of the in vitro diagnostic reagent product is carried out using the same technology; and store in a database research and development data of each research and development company obtained using research and development equipment for the research and development of the in vitro diagnostic reagent product according to the same research and development process, and
wherein the database is configured to allocate a storage space with the same structure to each of the research and development companies, and the research and development data of each of the research and development companies has data compatibility such that the research and development data is compared with each other.

2. The device for managing research and development of the in vitro diagnostic reagent product of claim 1, wherein the research and development management program provides the research and development companies with reference data for research and development extracted at least partially based on the research and development data stored in the database.

3. The device for managing research and development of the in vitro diagnostic reagent product of claim 1, wherein the same technology is at least one technology selected from the group consisting of a target gene sequence and amplicon determination technology, a candidate oligonucleotides design technology for a primer and a probe, an oligonucleotide structure technology, a signal generation mechanism indicating presence of a target, a technology for differentiating multiple signals generated from one label in one channel, a signal processing technology, a positive/negative determination technology, and a nucleic acid extraction technology.

4. The device for managing research and development of the in vitro diagnostic reagent product of claim 1, wherein the same research and development process includes at least one stage selected from the group consisting of a planning stage, a research and development stage, a release preparation stage, and a mass production transfer stage of the in vitro diagnostic reagent product.

5. The device for managing research and development of the in vitro diagnostic reagent product of claim 2, wherein the reference data for research and development is extracted from the research and development data of one or more of the research and development companies, and has the data compatibility such that the reference data for research and development is used in the research and development equipment of other research and development companies.

6. The device for managing research and development of the in vitro diagnostic reagent product of claim 1, wherein the data compatibility is standardized such that the data types of the research and development data are identical to each other, and the data formats are identical to each other.

7. The device for managing research and development of the in vitro diagnostic reagent product of claim 1, wherein the research and development data is at least one selected from the group consisting of base sequence data of a specific target, candidate oligonucleotides design data used for a nucleic acid amplification reaction experiment of a specific target, amplification curve data obtained from a nucleic acid amplification reaction of a specific target, amplification curve slope data, Ct value data, end RFU data, end RFU reading data, amplification efficiency data, sensitivity and specificity data, exclusivity and inclusivity, and setting values of analysis software and positive/negative determination data.

8. The device for managing research and development of the in vitro diagnostic reagent product of claim 1, wherein the research and development data includes a traceable data log, and the data log includes at least one selected from the group consisting of date, time, device, user and change contents in generation, viewing, output, modification and/or deletion of the research and development data.

9. The device for managing research and development of the in vitro diagnostic reagent product of claim 2, wherein the research and development data includes trouble data generated during the research and development of the in vitro diagnostic reagent product and trouble shooting data corresponding to the trouble data, and the reference data for research and development further includes the trouble shooting data.

10. The device for managing research and development of the in vitro diagnostic reagent product of claim 1, wherein the research and development data is further generated from a research and development support module included in the device for managing research and development and/or a research and development support device connected to the device for managing research and development via a communication network.

11. The device for managing research and development of the in vitro diagnostic reagent product of claim 1, wherein the research and development data is used to update performance of at least one of the research and development management program, the same technology, the research and development process, software used in the research and development equipment and the database, and wherein the software includes at least one of operation software and analysis software for the research and development equipment.

12. The device for managing research and development of the in vitro diagnostic reagent product of claim 1, wherein the research and development equipment includes at least one selected from the group consisting of a nucleic acid extraction device, a liquid handler, and a real-time PCR device.

13. The device for managing research and development of the in vitro diagnostic reagent product of claim 12, wherein the research and development equipment is the same equipment, and the same equipment is calibrated with the same calibration and/or operated by the same operation software.

14. The device for managing research and development of the in vitro diagnostic reagent product of claim 12, wherein the research and development equipment is the same equipment, and the same equipment is operated by the same operation software.

15. The device for managing research and development of the in vitro diagnostic reagent product of claim 12, wherein the research and development data is generated from the same operation software and the same analysis software for the same typed equipment.

16. The device for managing research and development of the in vitro diagnostic reagent product of claim 15, wherein the analysis software includes at least one selected from the group consisting of a baselining algorithm, an amplification curve fitting algorithm, a Ct value determination algorithm, a melting curve analysis algorithm, and a positive/negative determination algorithm.

17. The device for managing research and development of the in vitro diagnostic reagent product of claim 1, wherein the research and development management program provides the same research and development process that guides direction and strategy for research and development of the in vitro diagnostic reagent product; and
wherein the research and development management program provides at least one selected from the group consisting of the same technology, research and development equipment, software, an algorithm, a research and development plan, a candidate oligonucleotides design, an experimental process and condition for a performance test on oligonucleotide, a clinical sample, consumables, an enzyme, a nucleic acid extraction method, an extraction reagent and a protocol for nucleic acid amplification, or instruction information corresponding thereto.

18. The device for managing research and development of the in vitro diagnostic reagent product of claim 1, wherein the research and development process is used by the research and development companies to research and develop a plurality of the same in vitro diagnostic reagent products using the same raw material.

19. The device for managing research and development of the in vitro diagnostic reagent product of claim 1, wherein the same research and development process is a standardized research and development process provided by the device for managing research and development of in vitro diagnostic reagent product.

20. The device for managing research and development of the in vitro diagnostic reagent product of claim 19, wherein the research and development companies partially use a non-standard research and development process, and the non-standard research and development process includes at least one stage selected from the group consisting of a planning stage, a research and development stage, a release preparation stage, and a mass production transfer stage of the in vitro diagnostic reagent product.

21. The device for managing research and development of the in vitro diagnostic reagent product of claim 20, wherein non-standard research and development data generated at any stage corresponding to the non-standard research and development process is stored in the database, and
wherein the device for managing research and development of in vitro diagnostic reagent product further includes the non-standard research and development process in the standardized research and development process when the non-standard research and development process obtains a result determined to be a standardization target at least partially based on the non-standard research and development data.

22. The device for managing research and development of the in vitro diagnostic reagent product of claim 1, wherein the memory further stores a production management program that is provided to the research and development companies producing the in vitro diagnostic reagent product, and
wherein the production management program further provides the research and development companies with the same production process producing the in vitro diagnostic reagent products and further stores production data obtained using production equipment for producing the in vitro diagnostic reagent product in the database, and the production data of each of the research and development companies has the data compatibility such that the production data is compared with each other.

23. The device for managing research and development of the in vitro diagnostic reagent product of claim 22, wherein the production management program provides the research and development companies with reference data for research and development extracted at least partially based on the production data stored in the database.

24. The device for managing research and development of the in vitro diagnostic reagent product of claim 22, wherein the production data is at least one data selected from the group consisting of usage data of a raw material for producing the reagent products, inventory data of the raw material, inbound/outbound data of the raw material, trouble data generated during production, trouble shooting data, manufacturing number data, production schedule data by item of the reagent products, production volume data, usage data of consumables for producing the reagent products, inventory data of the consumables, inbound/outbound data of the consumables, and voice of the consumer (VoC) data.

25. The device for managing research and development of the in vitro diagnostic reagent product of claim 22, wherein the production data further includes raw material inventory information of the research and development companies producing the in vitro diagnostic reagent product through the same production process and/or product inventory information for the produced in vitro diagnostic reagent product.

26. The device for managing research and development of the in vitro diagnostic reagent product of claim 22, wherein the same production process includes at least one stage selected from the group consisting of a mass production stage, a release stage, and a post-management stage of the in vitro diagnostic reagent product.

27. The device for managing research and development of the in vitro diagnostic reagent product of claim 25, wherein the production data includes quality control information (QC information) for quality control performed for each of at least one selected stage among the same production processes, and the quality control information is generated by each research and development company receiving the production management program using the same quality control tools.

28. The device for managing research and development of the in vitro diagnostic reagent product of claim 22, wherein the data compatibility is standardized such that data types of the production data are identical to each other and data formats are identical to each other.

29. The device for managing research and development of the in vitro diagnostic reagent product of claim 22, wherein the research and development management program and the production management program are selectively provided to each research and development company among the research and development companies depending on whether the research and development business and the production business of the in vitro diagnostic reagent product are being performed.

30. A computer-implemented method for research and development of in vitro diagnostic reagent product which is performed in a device for managing research and development of in vitro diagnostic reagent product using a memory, a processor, and one or more programs stored in the memory and configured to be executed by the processor,
wherein the device for managing research and development stores a research and development management program that is provided to research and development companies developing the in vitro diagnostic reagent product,
wherein the research and development management program includes: commonly providing the same research and development process to the research and development companies such that the research and development of the in vitro diagnostic reagent product is executed using the same technology; and
storing in a database the research and development data of each research and development company obtained using the research and development equipment for the research and development of the in vitro diagnostic reagent product according to the same research and development process, and
wherein the database is configured to allocate a storage space with the same structure to each of the research and development companies, and the research and development data of each of the research and development companies has data compatibility such that the research and development data is compared with each other.

31. A computer-readable recording medium storing a computer program allowing research and development companies to manage research and development of in vitro diagnostic reagent product by a device for managing research and development of in vitro diagnostic reagent product,
wherein the computer program performs: commonly providing the same research and development process to the research and development companies such that the research and development of the in vitro diagnostic reagent product is carried out using the same technology; and
storing in a database the research and development data of each research and development company obtained using the research and development equipment for the research and development of the in vitro diagnostic reagent product according to the same research and development process, and
wherein the database is configured to allocate a storage space with the same structure to each of the research and development companies, and the research and development data of each of the research and development companies has data compatibility such that the research and development data is compared with each other.

32. A computer program stored on a computer-readable recording medium for allowing research and development companies to manage research and development of in vitro diagnostic reagent product by a device for managing research and development of in vitro diagnostic reagent product,
wherein the computer program is programmed to perform commonly providing the same research and development process to the research and development companies such that the research and development of the in vitro diagnostic reagent product is carried out using the same technology; and
storing in a database the research and development data of each research and development company obtained using the research and development equipment for the research and development of the in vitro diagnostic reagent product according to the same research and development process, and
wherein the database is configured to allocate a storage space with the same structure to each of the research and development companies, and the research and development data of each of the research and development companies has data compatibility such that the research and development data is compared with each other.
